(19) Europäisches Patentamt
European Patent Office
Office européen des brevets

(11) EP 4 647 494 A1

(12) **EUROPEAN PATENT APPLICATION**
published in accordance with Art. 153(4) EPC

(43) Date of publication:
**12.11.2025 Bulletin 2025/46**

(21) Application number: **24752720.3**

(22) Date of filing: **29.01.2024**

(51) International Patent Classification (IPC):
***C12N 9/74*** *(2006.01)* ***C07K 7/04*** *(2006.01)*
***C07K 14/00*** *(2006.01)* ***A61K 38/16*** *(2006.01)*
***A61P 31/04*** *(2006.01)*

(52) Cooperative Patent Classification (CPC):
**A61K 38/16; A61K 38/48; A61P 29/00; A61P 31/04; C07K 7/04; C07K 14/00; C12N 9/6429**

(86) International application number:
**PCT/CN2024/074412**

(87) International publication number:
**WO 2024/164864 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **07.02.2023 CN 202310070521**

(71) Applicant: **Hunan Jiudian Pharmaceutical Co., Ltd**
**Changsha, Hunan 410006 (CN)**

(72) Inventors:
- **LI, Yilong**
**Zhuzhou, Hunan 412000 (CN)**
- **YANG, Tiping**
**Zhuzhou, Hunan 412000 (CN)**
- **WANG, Deng**
**Zhuzhou, Hunan 412000 (CN)**
- **LIU, Huiqing**
**Zhuzhou, Hunan 412000 (CN)**
- **WANG, Xinbo**
**Zhuzhou, Hunan 412000 (CN)**
- **GONG, Huanzhang**
**Zhuzhou, Hunan 412000 (CN)**
- **LI, Xiangqun**
**Zhuzhou, Hunan 412000 (CN)**

(74) Representative: **HGF**
**HGF Limited**
**1 City Walk**
**Leeds LS11 9DX (GB)**

Remarks:
The complete document including Reference Table(s) and the Sequence Listing(s) can be downloaded from the EPO website

(54) **POLYPEPTIDE AND USE THEREOF**

(57) The invention belongs to the technical field of biomedical polypeptides, and particularly relates to a polypeptide and a use thereof in preparation of a drug for preventing, managing, treating or alleviating an antibacterial and/or anti-inflammatory related disease. The polypeptide of the invention has a short amino acid sequence and a simple structure, is convenient to prepare, has a high antibacterial/anti-inflammatory activity, can be used for preventing and treating a disease caused by microbial infection, and has a good healing effect on a skin wound.

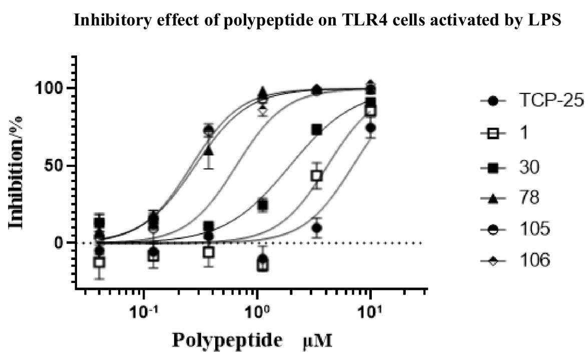


FIG. 11

EP 4 647 494 A1

## Description

### TECHNICAL FIELD

The present invention belongs to the technical field of biomedical polypeptides, and particularly relates to a polypeptide and a use thereof in preparation of a drug for preventing, managing, treating or alleviating an antibacterial and/or anti-inflammatory related disease.

### BACKGROUND

Skin wound generally refers to a direct injury caused by an external force or an injury caused by soft tissue infection, which will cause local inflammation, local redness and swelling, pain and suppuration, or other symptom. After common skin injuries occur, traditional inflammatory symptoms (redness, swelling and fever) are caused by the leakage of intravascular liquid and blood and the obstruction of local lymphatic drainage. Due to cell injury, some vasoactive substances, such as histamine, serotonin and catecholamine, are released, which cause temporary vasoconstriction, followed by vasodilation, thus allowing liquids and cells to enter an extravascular injury site. In clinic, most of the existing treatment means adopt antibiotics, and the antibiotics have become the most widely used drugs for skin injury. However, with the extensive application of the antibiotics, the drug resistance of bacteria has become the main problem of such treatment means, and meanwhile, bacterial infection may cause an inflammatory reaction of human body, which will also delay skin healing and lead to the existence of wound scar. The common skin injuries, such as scalds, burns and ulcers, in daily life are mainly managed by antibacterial means, and are not properly treated, so that there is a clinical need that has not been met yet.

Therefore, a new antibacterial/anti-inflammatory drug has become a research and development hot spot in the fields of human medicine, nutrition, food science and immunology at home and abroad, and the development of biological antibacterial peptides is expected to solve the problem of antimicrobial resistance. Meanwhile, the antibacterial peptides with an anti-inflammatory effect will effectively meet the clinical need in the field of skin injuries, thus having broad development and application prospects.

A human TCP (Thrombin-derived C-terminal peptide) is initially a Thrombin-derived C-terminal peptide with a size of about 2 kDa obtained by selectively hydrolyzing blood coagulation factors, and a sequence of the peptide has a length of 25 amino acids and contains two action targets. The peptide can not only interact with LPS on a bacterial surface, but also bind with a hydrophobic pocket on a human leukocyte surface differentiation antigen CD14, thus playing an anti-endotoxin function in vivo and in vitro, and preventing shock and other problems caused by sepsis. Existing researches show that the TCP-25 can effectively inhibit Staphylococcus aureus and Gram-negative Pseudomonas aeruginosa and subcutaneously infectious Pseudomonas in vitro, so that the response of macrophages to serum lipase (LPS) in vivo is reduced, and there are certain therapeutic potentials in promoting wound healing and reducing scar hyperplasia. Therefore, the TCP-25 is a polypeptide with a dual-target action mechanism, which can resist bacteria and inflammation simultaneously, thus having the advantages in specificity, clinical efficacy and toxic and side effects. Based on the dual action mechanism, high broad-spectrum bacteriostasis, low toxicity and other characteristics of the TCP-25, the present invention provides an antibacterial/anti-inflammatory polypeptide modified based on the TCP-25, so that it is expected to develop a promising drug for wound healing.

### SUMMARY

The following only generally describes some aspects of the present invention, but the present invention is not limited by this. These aspects and other parts are explained more completely later. All references in the specification are hereby incorporated by reference in their entirety. In case of any discrepancy between the disclosed contents in the specification and the references, the disclosed contents in the specification shall prevail.

The present invention aims to provide a novel antibacterial and/or anti-inflammatory polypeptide to solve the problem of unmet clinical need in skin wound treatment.

An alanine scanning technology refers to, by utilizing the characteristic that alanine is small in size and has little influence on a structure of a protein, but some functions of the protein can be weakened or declined when other key amino acids are replaced with the alanine, replacing other 19 non-alanine residues with the alanine one by one, constructing an alanine scanning library, and identifying an important role of a certain amino acid residue in the function, active site, stability and morphology of the protein through expression and screening. Alanine scanning is carried out on a sequence of TCP-25, and then antibacterial and anti-inflammatory activities of molecules are detected, which are compared with activities of the sequence of TCP-25 before mutation, so as to determine amino acid sites important for the activities. According to the obtained information, single amino acid mutation and multiple amino acid combination mutation are further carried out at some sites, and finally, a series of antibacterial and/or anti-inflammatory peptides are obtained.

Specifically, in a first aspect, the present invention provides a polypeptide, comprising a sequence as shown in formula (I) below:

$$(X^0)_n\text{-}X^1\text{-}X^2\text{-}X^3\text{-}X^4\text{-}L\text{-}X^5\text{-}K\text{-}W\text{-}I\text{-}X^6\text{-}K\text{-}X^7\text{-}X^8\text{-}(X^9)_m \qquad (I)$$

or a pharmaceutically acceptable salt thereof, wherein:

$X^0$ is independently selected from any amino acid;
$X^1$, $X^4$ and $X^5$ are respectively independently selected from K, dK, R, dR, and a derivative of K or R;
$X^2$, $X^3$ and $X^6$ are respectively independently selected from any amino acid;
$X^7$ and $X^8$ are respectively independently selected from I, V, L, T, or the d configuration of the amino acid and the derivative thereof;
each $X^9$ is independently any amino acid;
n is 0 or an integer of 1-7; and
m is 0 or an integer of 1-5.

In some embodiments, the X° is selected from G, K, Y, F, T, S, K, L, W, E, or the d configuration of the amino acid and the derivative thereof.

In some embodiments, the $X^2$ is selected from V, L, T, K, W, E, or the d configuration of the amino acid and the derivative thereof.

In some embodiments, the $X^3$ is selected from F, Y, W, or the d configuration of the amino acid and the derivative thereof.

In some embodiments, the $X^6$ is selected from Q, N, T, K, W, L, or the d configuration of the amino acid and the derivative thereof.

In some embodiments, the $X^1$ is selected from Lys, $^D$Lys, Orn, $^D$Orn, Dab, $^D$Dab, hLys, $^D$hLys, ACLys, $^D$ACLys, NMeLys, NMe$^D$Lys, NMeDab or NMe$^D$Dab.

In some embodiments, the $X^9$ is independently selected from C, Q, F, G, N, T, K, W, L, Y, S, D, E, or the d configuration of the amino acid and the derivative of the amino acid above; and preferably, the $X^9$ is selected from Phe, $^D$Phe, Trp, $^D$Trp, Tyr, $^D$Tyr, Thi, $^D$Thi, Bip, $^D$Bip, Nva, $^D$Nva, Aib, $^D$Aib, Pip, $^D$Pip, hPhe, $^D$hPhe, hTrp, $^D$hTrp, hTyr, $^D$hTyr, hHis, $^D$hHis, 1-Nal, 2-Nal, Dip, 1-$^D$Nal, 2-$^D$Nal and $^D$Dip.

In some embodiments, when m=5, first and last amino acids of $(X^9)_m$ are linked into a ring through a bond; preferably, the bond is selected from a disulfide bond, an amide bond, a carbon-hydrogen bond, dithioamino $C_{1-6}$ alkyl, a thioether bond or an olefin thioether bond; and more preferably, the bond is one of the following structures:

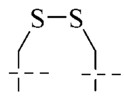, 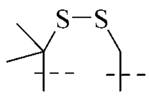,

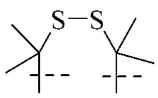, 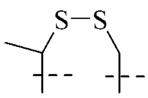, 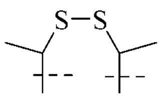, 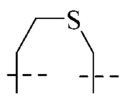, 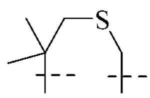, 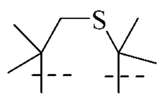, 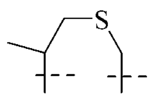,

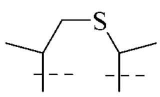, 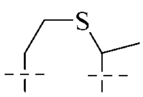, 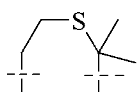, 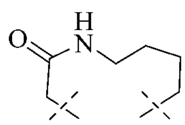, 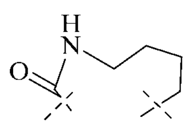, 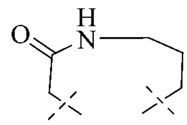,

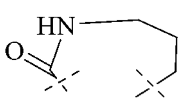, 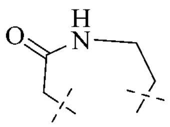, 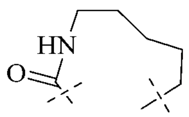, 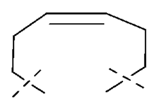, 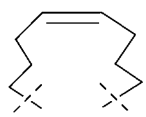, 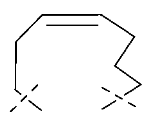,

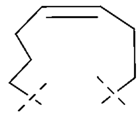, 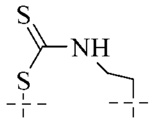 and 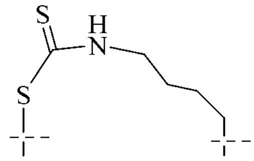.

In another aspect, the present invention provides a polypeptide, comprising a sequence as shown in formula (II) below:

$$G\text{-}K\text{-}X^{10}\text{-}G\text{-}X^{11}\text{-}Y\text{-}X^{12}\text{-}X^{1}\text{-}X^{2}\text{-}X^{3}\text{-}X^{4}\text{-}L\text{-}X5\text{-}K\text{-}W\text{-}I\text{-}X^{6}\text{-}K\text{-}X^{7}\text{-}X^{8}\text{-}(X^{9})_{m} \qquad \text{(II)}$$

or a pharmaceutically acceptable salt thereof, wherein:

$X^1$, $X^4$ and $X^5$ are respectively independently selected from K, dK, R, dR, and a derivative of K or R;
$X^2$, $X^3$, $X^6$, $X^{11}$ and $X^{12}$ are respectively independently selected from any amino acid;
$X^7$ is selected from V, L, or a d configuration of the amino acid and a derivative thereof;
$X^8$ is selected from I, V, L, T, or the d configuration of the amino acid and the derivative thereof;
each $X^9$ is independently any amino acid;
$X^{10}$ is an optional aromatic amino acid;
m is 0 or an integer of 1-5.

In some embodiments, the $X^2$ is selected from V, L, T, K, W, E, or the d configuration of the amino acid and the derivative thereof.

In some embodiments, the $X^3$ and the $X^{11}$ are respectively independently selected from F, Y, W, or the d configuration of the amino acid and the derivative thereof.

In some embodiments, the $X^6$ is selected from Q, N, T, K, W, L, or the d configuration of the amino acid and the derivative thereof.

In some embodiments, the $X^{12}$ is T, S, K, L, W, E, or the d configuration of the amino acid and the derivative thereof.

In some embodiments, the $X^9$ is selected from C, Q, F, G, N, T, K, W, L, Y, S, D, E, or the d configuration of the amino acid and the derivative of the amino acid above; and preferably, the $X^9$ is selected from Phe, $^{D}$Phe, Trp, $^{D}$Trp, Tyr, $^{D}$Tyr, Thi, $^{D}$Thi, Bip, $^{D}$Bip, Nva, $^{D}$Nva, Aib, $^{D}$Aib, Pip, $^{D}$Pip, hPhe, $^{D}$hPhe, hTrp, $^{D}$hTrp, hTyr, $^{D}$hTyr, hHis, $^{D}$hHis, 1-Nal, 2-Nal, Dip, 1-$^{D}$Nal, 2-$^{D}$Nal and $^{D}$Dip.

In some embodiments, the $X^{10}$ is Y, F, W, dY, dF, dW, or the derivative of the amino acid above; and preferably, the $X^{10}$ is selected from Phe, $^{D}$Phe, Trp, $^{D}$Trp, Tyr, $^{D}$Tyr, Thi, $^{D}$Thi, Bip, $^{D}$Bip, hPhe, $^{D}$hPhe, hTrp, $^{D}$hTrp, hTyr, $^{D}$hTyr, hHis, $^{D}$hHis, 1-Nal, 2-Nal, Dip, 1-$^{D}$Nal, 2-$^{D}$Nal or $^{D}$Dip.

In some embodiments, when m=5, first and last amino acids of $(X^9)_m$ are linked into a ring through a bond; preferably, the bond is selected from a disulfide bond, an amide bond, a carbon-hydrogen bond, dithioamino $C_{1-6}$ alkyl, a thioether bond or an olefin thioether bond; and more preferably, the bond is one of the following structures:

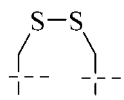, 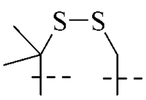,

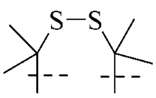, 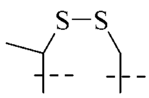, 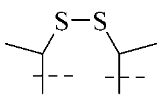, 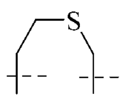, 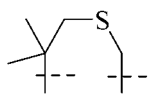, 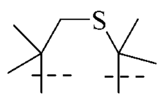, 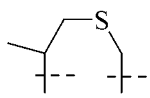,

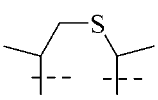, 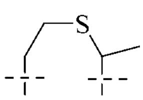, 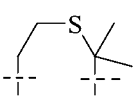, 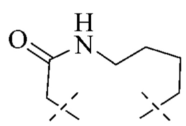, 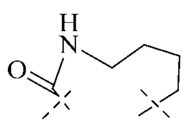, 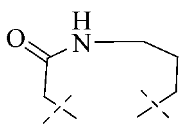,

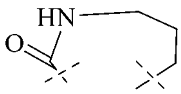, 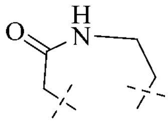, 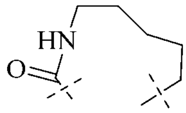, 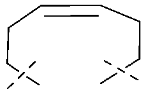, 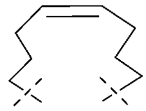, 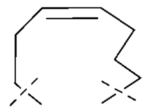,

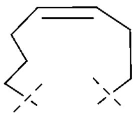, 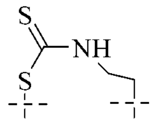 and 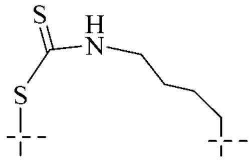.

In another aspect, the present invention provides a polypeptide, comprising a sequence as shown in formula (III) below:

$$G\text{-}K\text{-}X^{10}\text{-}G\text{-}X^{11}\text{-}Y\text{-}X^{12}\text{-}X^{1}\text{-}X^{2}\text{-}X^{3}\text{-}X^{4}\text{-}L\text{-}X^{5}\text{-}K\text{-}W\text{-}I\text{-}X^{6}\text{-}K\text{-}X^{7}\text{-}X^{8}\text{-}X^{13}\text{-}X^{14}\text{-}X^{15}\text{-}G\text{-}X^{16} \qquad \text{(III)}$$

or a pharmaceutically acceptable salt thereof, wherein:

$X^1$, $X^4$ and $X^5$ are respectively independently selected from K, dK, R, dR, and a derivative of K or R;
$X^2$, $X^3$, $X^6$, $X^{10}$, $X^{11}$, $X^{12}$, $X^{13}$, $X^{14}$, $X^{15}$ and $X^{16}$ are respectively independently selected from any amino acid;
$X^7$ is selected from V, L, or a d configuration of the amino acid and a derivative thereof;
$X^8$ is selected from I, V, L, T, or the d configuration of the amino acid and the derivative thereof.

In some embodiments, the $X^2$ is selected from V, L, T, K, W, E, or the d configuration of the amino acid and the derivative thereof.

In some embodiments, the $X^3$ and the $X^{11}$ are respectively independently selected from F, Y, W, or the d configuration of the amino acid and the derivative thereof.

In some embodiments, the $X^6$ is selected from Q, N, T, K, W, L, or the d configuration of the amino acid and the derivative thereof.

In some embodiments, the $X^{10}$ is Y, F, W, or the d configuration of the amino acid and the derivative thereof.

In some embodiments, the $X^{12}$ is T, S, K, L, W, E, or the d configuration of the amino acid and the derivative thereof.

In some embodiments, the $X^{13}$ and the $X^{16}$ are respectively independently selected from C, K, W, L, S, D, E, or the d configuration of the amino acid and the derivative thereof; and preferably, the $X^{13}$ and the $X^{16}$ are respectively independently selected from C or d-C.

In some embodiments, the $X^{13}$ and the $X^{16}$ are linked into a ring through a bond; preferably, the bond is selected from a disulfide bond, an amide bond, a carbon-hydrogen bond, dithioamino $C_{1\text{-}6}$ alkyl, a thioether bond or an olefin thioether bond; and more preferably, the bond is one of the following structures:

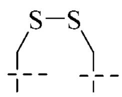, 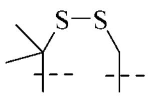, 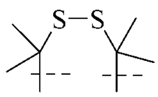, 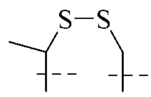,

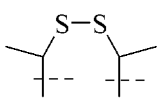, 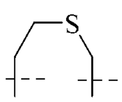, 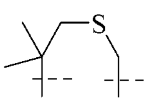, 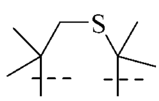, 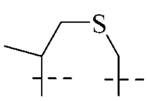, 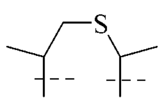, 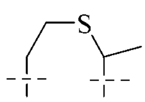,

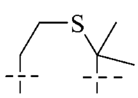, 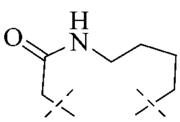, 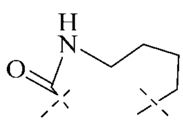, 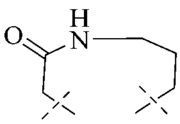, 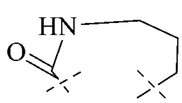, 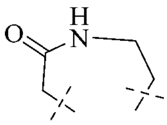,

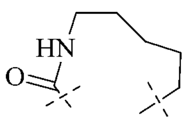 , 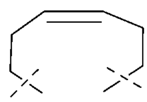 , 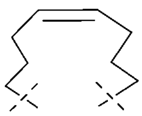 , 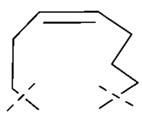 , 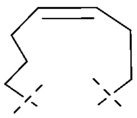 , 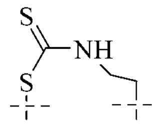

and

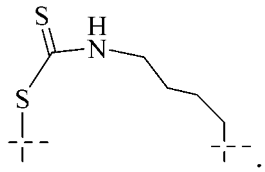

In some embodiments, the $X^{14}$ is Q, N, T, K, W, L, or the d configuration of the amino acid and the derivative thereof.

In some embodiments, the $X^{15}$ is Y, F, W, or the d configuration of the amino acid and the derivative thereof.

Preferably, the present invention provides a polypeptide with one of the following amino acids: SEQ ID No. 1-SEQ ID No. 99.

In another aspect, the present invention provides a polypeptide, and a sequence of the polypeptide is one of SEQ ID No. 1-SEQ ID No. 99; and preferably, the sequence of the polypeptide is selected from SEQ ID No. 1 (corresponding to a compound 1 hereinafter), SEQ ID No. 10 (corresponding to a compound 17 hereinafter), SEQ ID No. 23 (corresponding to a compound 30 hereinafter), SEQ ID No. 25 (corresponding to a compound 32 hereinafter), SEQ ID No. 30 (corresponding to a compound 37 hereinafter), SEQ ID No. 74 (corresponding to a compound 84 hereinafter), SEQ ID No. 76 (corresponding to a compound 86 hereinafter), SEQ ID No. 78 (corresponding to a compound 88 hereinafter), SEQ ID No. 80 (corresponding to a compound 90 hereinafter), SEQ ID No. 95 (corresponding to a compound 105 hereinafter) or SEQ ID No. 96 (corresponding to a compound 106 hereinafter).

In another aspect, the present invention provides a polypeptide, and the polypeptide is one of the following structures or a pharmaceutically acceptable salt thereof, wherein a mark number below the sequence represents a serial number of compound, for example, 1 represents the compound 1, 2 represents the compound 2, and so on:

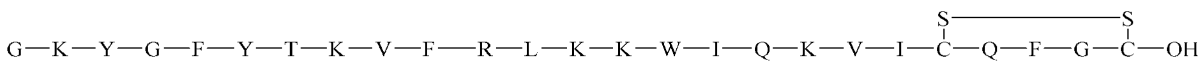

1

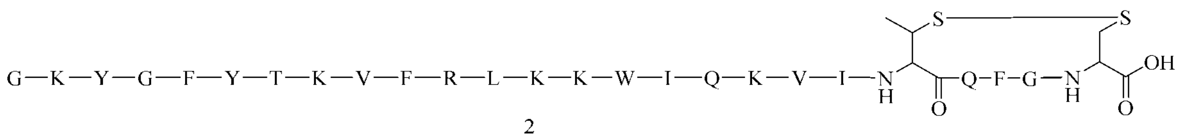

2

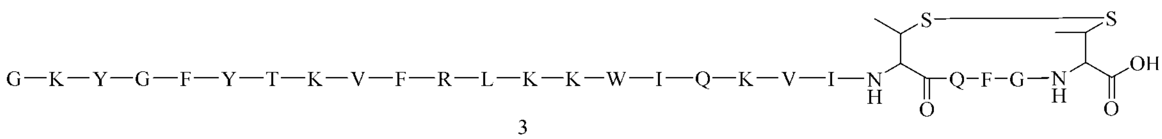

3

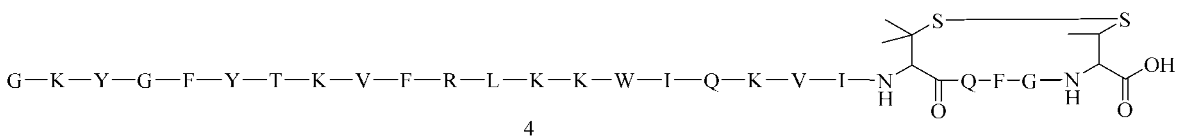

4

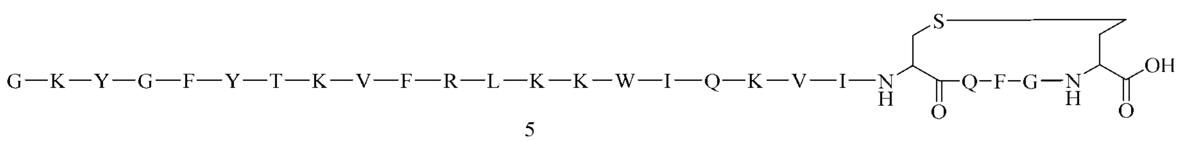

5

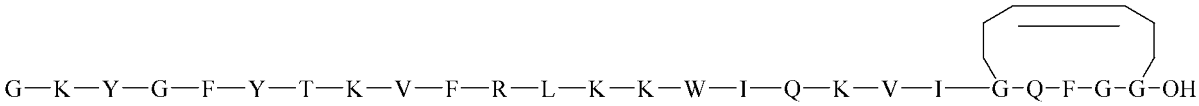

6

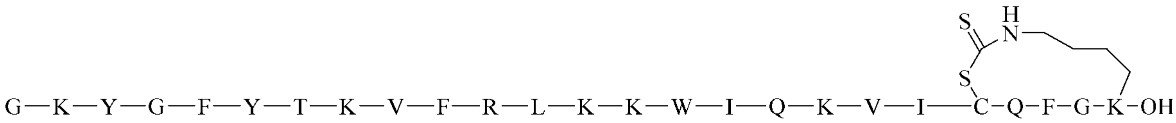

7

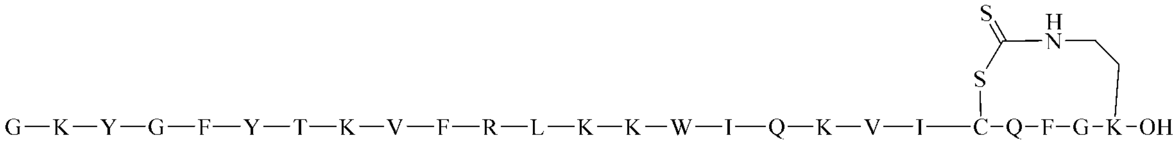

8

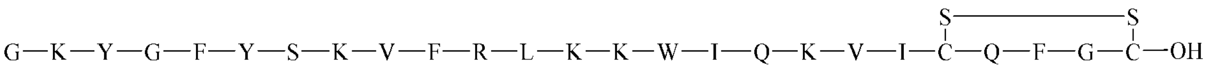

9

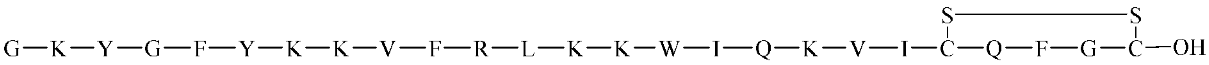

10

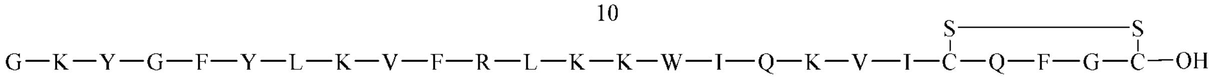

11

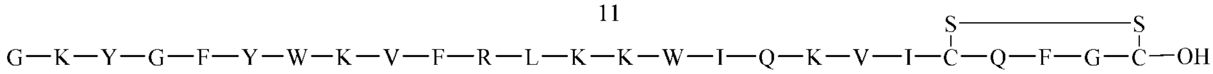

12

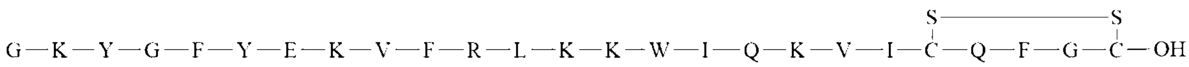

13

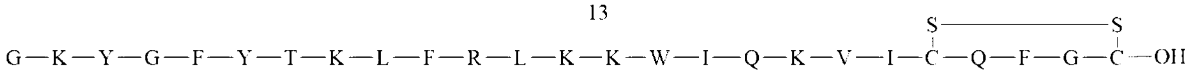

14

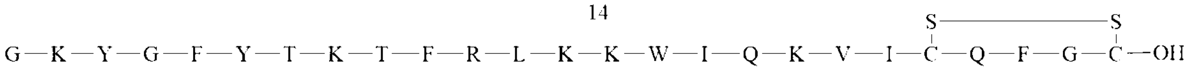

15

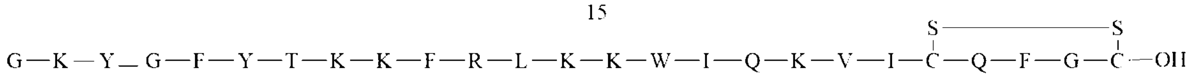

16

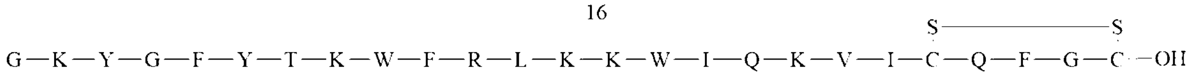

17

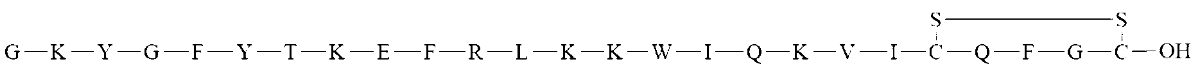

18

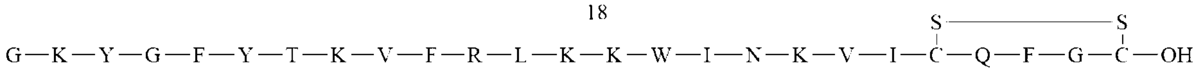

19

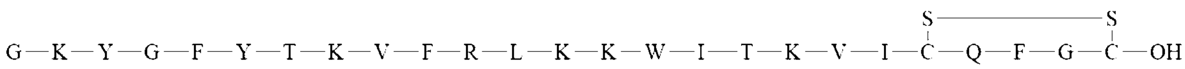

20

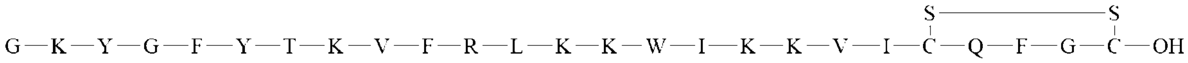

21

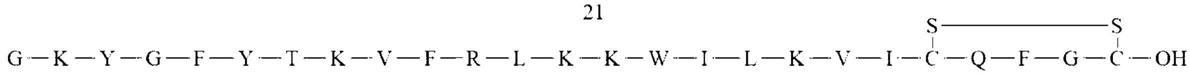

22

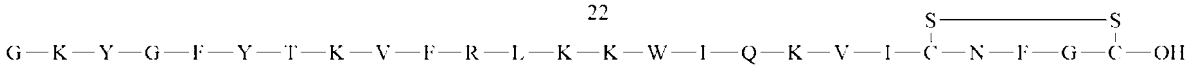

23

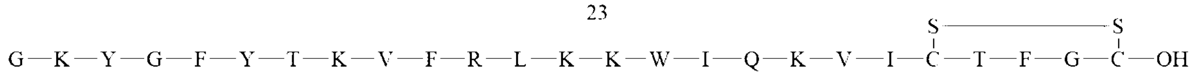

24

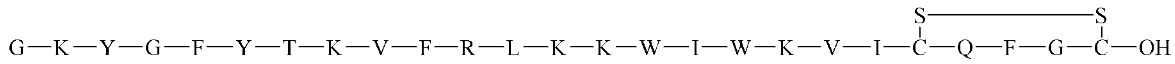

25

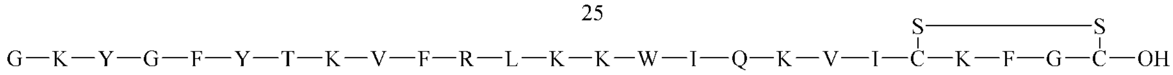

26

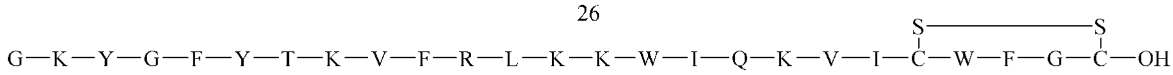

27

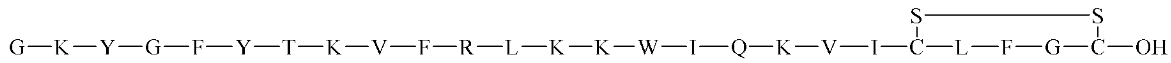

28

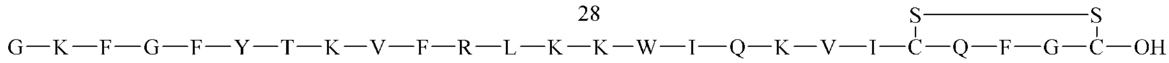

29

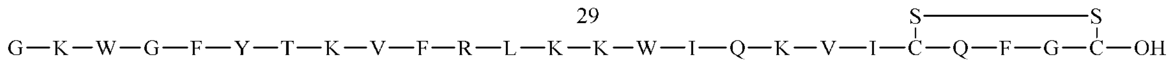

30

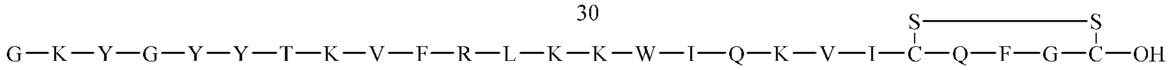

31

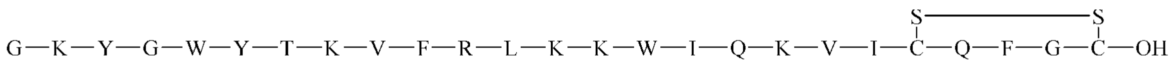

32

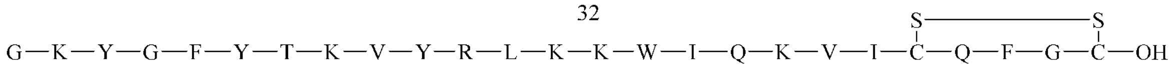

33

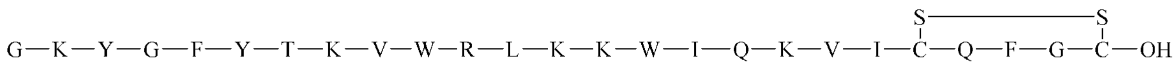

34

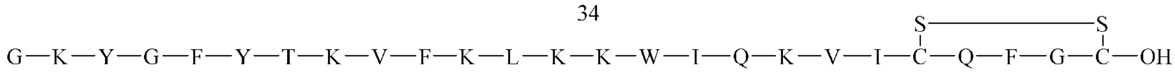

35

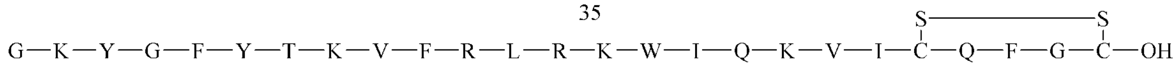

36

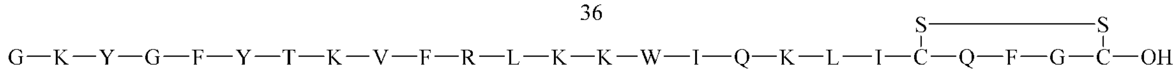

37

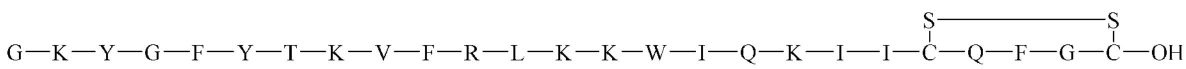

38

S—————S
G—K—Y—G—F—Y—T—K—V—F—R—L—K—K—W—I—Q—K—V—L—C—Q—F—G—C—OH

39

S—————S
G—K—Y—G—F—Y—T—K—V—F—R—L—K—K—W—I—Q—K—V—V—C—Q—F—G—C—OH

40

S—————S
G—K—Y—G—F—Y—T—K—V—F—R—L—K—K—W—I—Q—K—V—T—C—Q—F—G—C—OH

41

S—————S
G—K—Y—G—F—Y—T—K—V—F—R—L—K—K—W—I—Q—K—V—I—C—Q—Y—G—C—OH

42

S—————S
G—K—Y—G—F—Y—T—K—V—F—R—L—K—K—W—I—Q—K—V—I—C—Q—W—G—C—OH

43

S—————S
G—K—Y—G—F—Y—T—K—V—F—R—L—K—K—W—I—Q—K—V—I—dC—Q—F—G—C—OH

44

S—————S
G—K—Y—G—F—Y—T—K—V—F—R—L—K—K—W—I—Q—K—V—I—dC—Q—F—G—dC—OH

45

S—————S
G—K—Y—G—F—Y—T—K—V—F—R—L—K—K—W—I—Q—K—V—I—C—Q—F—G—dC—OH

46

G—K—Y—G—F—Y—T—K—V—F—R—L—K—K—W—I—Q—K—V—I—K—Q—F—G—E—OH

47

G—K—Y—G—F—Y—T—K—V—F—R—L—K—K—W—I—Q—K—V—I—D—Q—F—G—K—OH

48

G—K—Y—G—F—Y—T—K—V—F—R—L—K—K—W—I—Q—K—V—I—K—Q—F—G—K—OH

49

G—K—Y—G—F—Y—T—K—V—F—R—L—K—K—W—I—Q—K—V—I—L—Q—F—G—E—OH

50

G—K—Y—G—F—Y—T—K—V—F—R—L—K—K—W—I—Q—K—V—I—W—Q—F—G—E—OH

51

G—K—Y—G—F—Y—T—K—V—F—R—L—K—K—W—I—Q—K—V—I—S—Q—F—G—E—OH

52

G—K—Y—G—F—Y—T—K—V—F—R—L—K—K—W—I—Q—K—V—I—D—Q—F—G—L—OH

53

G—K—Y—G—F—Y—T—K—V—F—R—L—K—K—W—I—Q—K—V—I—D—Q—F—G—W—OH

54

G—K—Y—G—F—Y—T—K—V—F—R—L—K—K—W—I—Q—K—V—I—D—Q—F—G—S—OH

55

S ——— S

G—K—Y—G—F—Y—T—R—V—F—R—L—K—K—W—I—Q—K—V—I—C—Q—F—G—C—OH

56

S ——— S

G—K—W—G—W—Y—T—K—V—F—R—L—K—K—W—I—Q—K—V—I—C—Q—F—G—C—OH

57

S ——— S

G—K—W—G—W—Y—W—K—V—F—R—L—K—K—W—I—Q—K—V—I—C—Q—F—G—C—OH

58

S ——— S

G—K—W—G—F—Y—W—K—V—F—R—L—K—K—W—I—Q—K—V—I—C—Q—F—G—C—OH

59

S ——— S

G—K—W—G—W—Y—W—R—V—F—R—L—K—K—W—I—Q—K—V—I—C—Q—F—G—C—OH

60

S ——— S

G—K—W—G—W—Y—W—R—L—F—R—L—K—K—W—I—Q—K—V—I—C—Q—F—G—C—OH

61

S ——— S

G—K—W—G—W—Y—W—R—L—W—R—L—K—K—W—I—Q—K—V—I—C—Q—F—G—C—OH

62

S ——— S

G—K—W—G—W—Y—W—R—L—W—K—L—K—K—W—I—Q—K—V—I—C—Q—F—G—C—OH

63

S ——— S

G—K—W—G—W—Y—W—R—L—W—K—L—R—K—W—I—Q—K—V—I—C—Q—F—G—C—OH

64

S ——— S

G—K—W—G—W—Y—W—R—L—W—K—L—R—K—W—I—W—K—V—I—C—Q—F—G—C—OH

65

S ——— S

G—K—W—G—W—Y—W—R—L—W—K—L—R—K—W—I—L—K—V—I—C—Q—F—G—C—OH

66

S ——— S

G—K—W—G—W—Y—W—R—L—W—K—L—R—K—W—I—W—K—L—I—C—Q—F—G—C—OH

67

S ——— S

G—K—W—G—W—Y—W—R—L—W—K—L—R—K—W—I—W—K—L—L—C—Q—F—G—C—OH

68

S ——— S

G—K—W—G—W—Y—W—R—L—W—K—L—R—K—W—I—W—K—L—L—C—T—F—G—C—OH

69

S ——— S

G—K—W—G—W—Y—W—R—L—W—K—L—R—K—W—I—W—K—L—L—C—T—W—G—C—OH

70

S ——— S

G—K—W—G—W—Y—W—R—L—W—K—L—R—K—W—I—L—K—L—L—C—T—W—G—C—OH

71

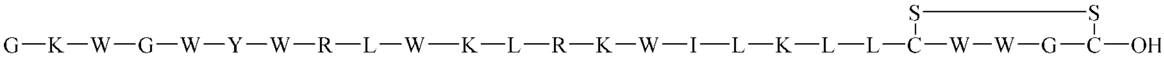

72

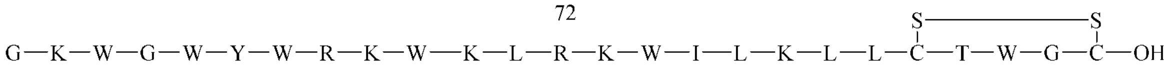

73

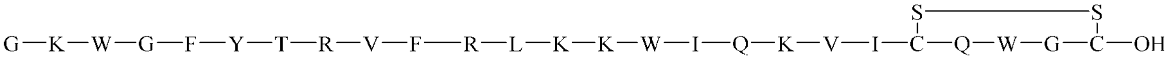

74

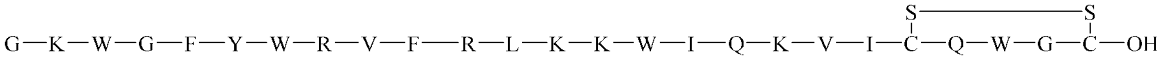

75

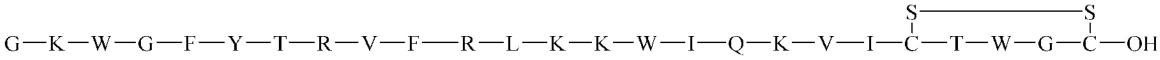

76

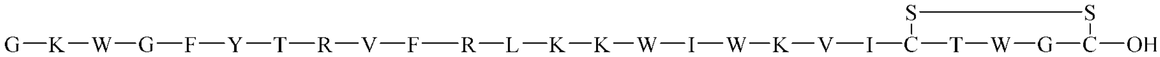

77

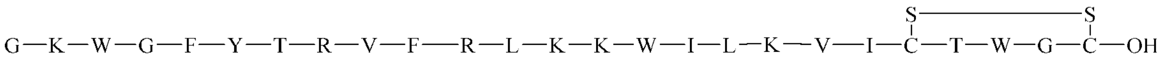

78

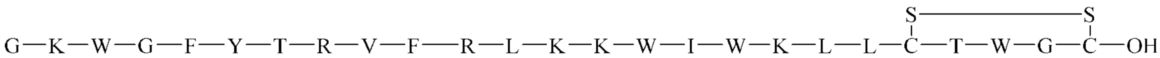

79

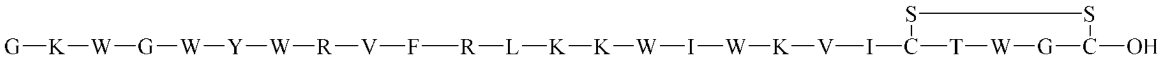

80

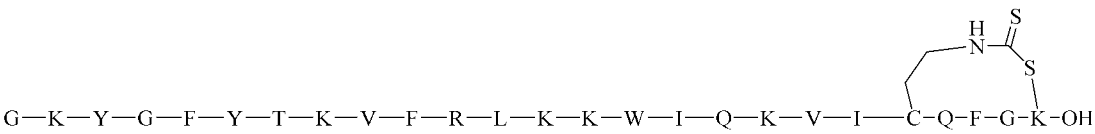

81

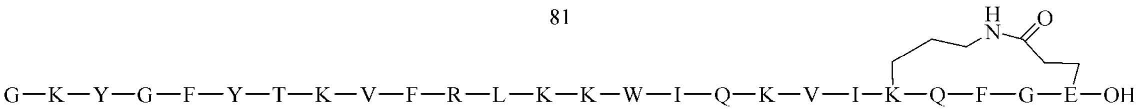

82

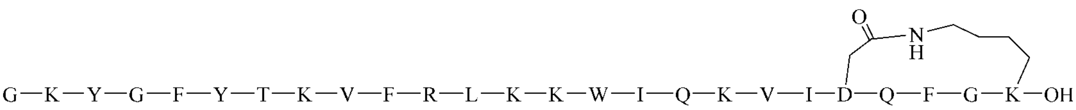

83

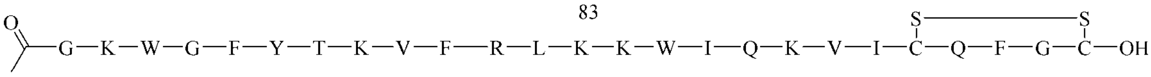

84

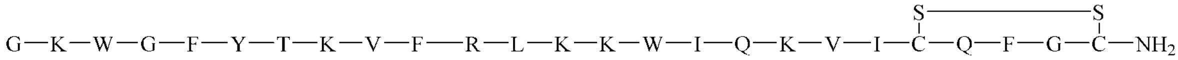

85

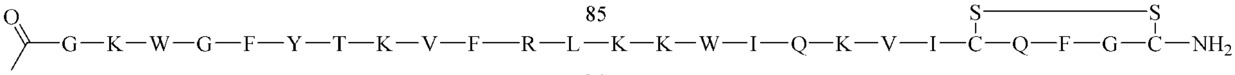

86

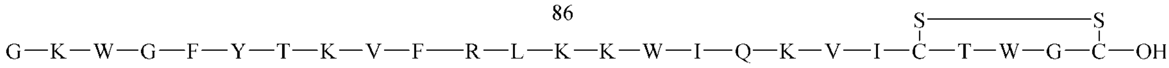

87

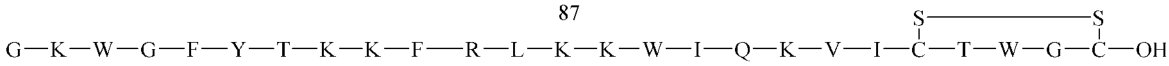

88

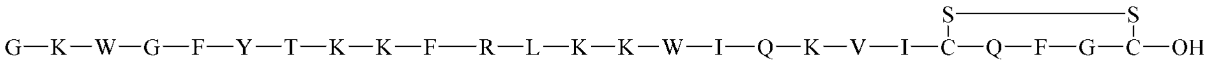

89

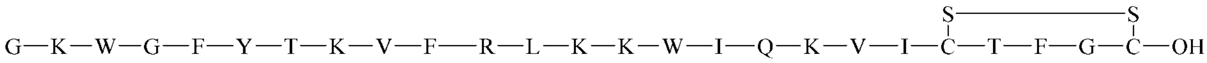

90

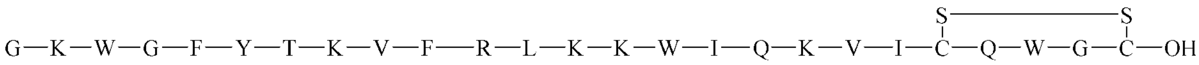

91

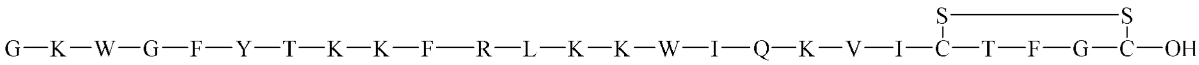

92

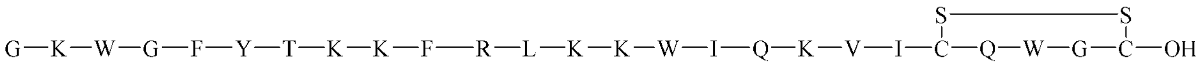

93

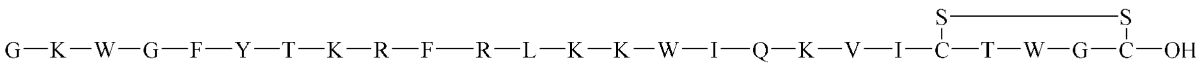

94

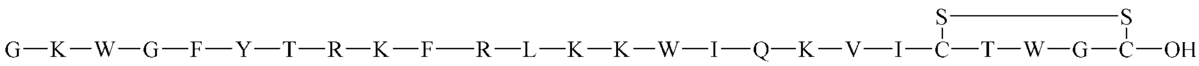

95

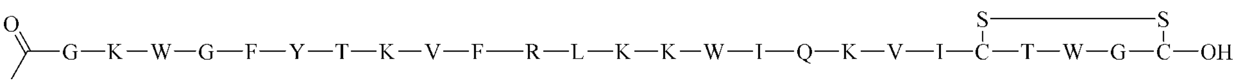

96

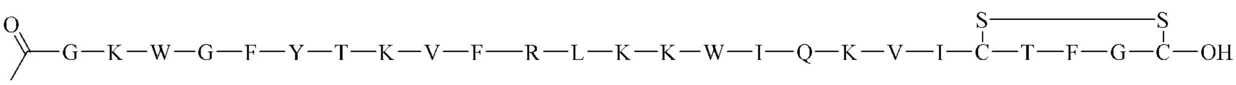

97

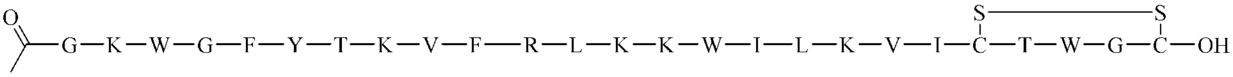

98

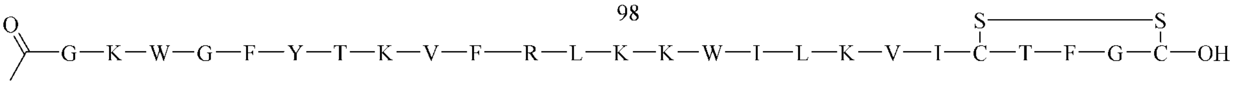

99

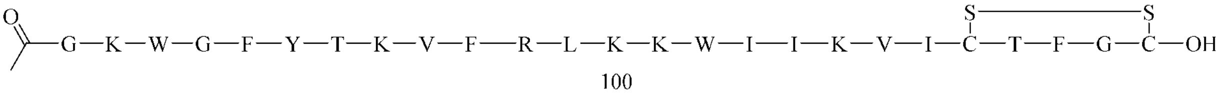

100

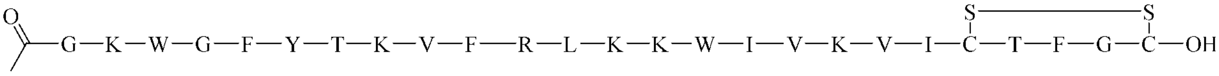

101

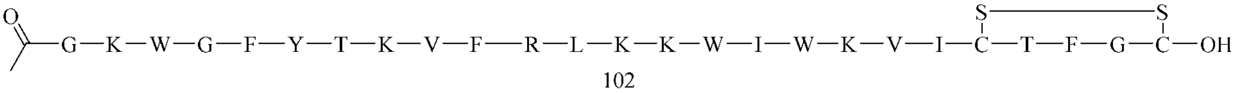

102

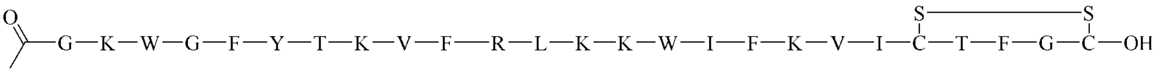

103

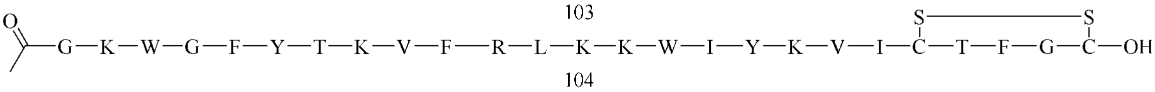

104

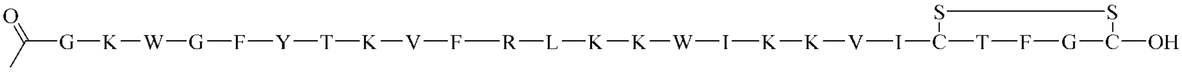

105

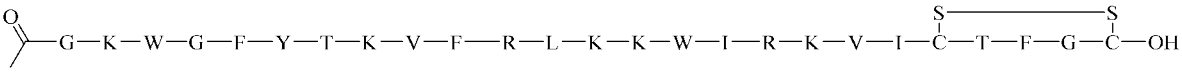

106

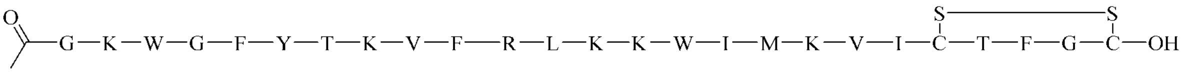

107

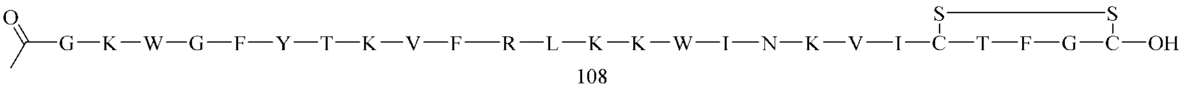

108

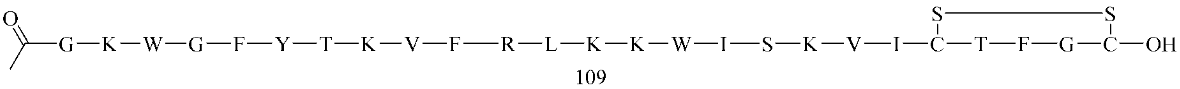

109

In one aspect, the present invention relates to a pharmaceutical composition, comprising any polypeptide of the present invention. In some embodiments, the pharmaceutical composition of the present invention further comprises at least one of a pharmaceutically acceptable carrier, an excipient, a diluent, an adjuvant and an intermedium.

In another aspect, the present invention relates to a use of the polypeptide or the pharmaceutical composition in preparation of a drug for preventing, managing, treating or alleviating a disease, wherein the drug is used for preventing, managing, treating or alleviating a bacterial infectious disease and/or an inflammatory infectious disease.

In some embodiments, the bacterial infectious disease and/or the inflammatory infectious disease comprises, but is not limited to, skin infection, skin wound infection, burn infection, scald infection, pneumonia, tuberculosis, diabetic foot, tonsillitis, bacillary dysentery, meningitis, scarlet fever, pharyngolaryngitis, bronchitis, gastritis, appendicitis, glomerulonephritis, endocarditis, pericarditis, cystitis, pelvic inflammation and cervicitis.

The present invention has the beneficial effects that: the polypeptide of the present invention has a significant antibacterial/anti-inflammatory effect, a high target affinity, a very low minimal inhibitory concentration (MIC) value and a large inhibition zone diameter; and a cyclic structure of the polypeptide of the present invention is beneficial for improving the stability of the polypeptide without producing obvious cytotoxicity to mammalian cells.

## BRIEF DESCRIPTION OF THE DRAWINGS

FIG. 1 is a MS graph of a compound 30;
FIG. 2 is a HPLC graph of the compound 30;
FIG. 3 is a MS graph of a compound 105;
FIG. 4 is a HPLC graph of the compound 105;
FIG. 5 is a MS graph of a compound 106;
FIG. 6 is a HPLC graph of the compound 106;
FIG. 7 is a MS graph of a compound 108;
FIG. 8 is a HPLC graph of the compound 108;
FIG. 9 is a MS graph of a compound 109;
FIG. 10 is a HPLC graph of the compound 109; and
FIG. 11 shows inhibitory effects of polypeptide compounds on expression of fluorescein reporter gene in TLR4 cells activated by LPS.

## DETAILED DESCRIPTION

The meaning of the term "peptide" or "polypeptide" is well known to those skilled in the art. In general, the peptide or the polypeptide consists of two or more amino acids linked through an amide bond, and the amide bond consists of amino of one amino acid and carboxyl of an adjacent amino acid. The polypeptide herein may contain naturally occurring amino acids or non-naturally occurring amino acids, which may be modified into an analogue thereof, a derivative thereof, a functional mimetic thereof, a pseudopeptide and other compounds containing at least two amino acids. Unless it is indicated that an N-terminal or a C-terminal has a specific modification, a polypeptide containing a specific amino acid sequence comprises unmodified and modified amino and/or carboxyl terminals, which is well known to those skilled in the art. The polypeptide with the specific amino acid sequence may comprise modified amino acids and/or additional amino acids, unless the N-terminal and/or the C-terminal contains a modification that hinders further addition of amino acids. Such modification comprises, for example, acetylation of the N-terminal and/or amidation of the C-terminal.

The polypeptide of the present invention may be modified to form a polypeptide derivative. As is well known to those skilled in the art, the polypeptide may be modified in various ways. Typical modifications comprise, but are not limited to, acetylation of the N-terminal, amidation of the C-terminal, d-configuration amino acid substitution, unnatural amino acid substitution, fatty acid modification or a combination of the above modifications. The present invention comprises any well-known modification to the polypeptide. For example, the polypeptide derivative may comprise a chemical modification to the polypeptide, such as alkylation, acylation, carbamylation, iodination or any other modification that produces the polypeptide derivative. The modification to the polypeptide may comprise modified amino acids, such as hydroxyproline or carboxyglutamic acid, and may comprise amino acids linked through a non-peptide bond.

For other modifications to the polypeptide of the present invention, unnatural amino acids may be used to substitute natural amino acids in the polypeptide, and the unnatural amino acids comprise, but are not limited to, 2-amino fatty acid (Aad), 3-amino fatty acid (βAad), β-alanine, β-aminopropionic acid (βAla), 2-aminobutyric acid (Abu), 4-aminobutyric acid, piperidinecarboxylic acid (4Abu), 6-aminocaproic acid (Acp), 2-aminoheptanoic acid (Ahe), 2-aminoisobutyric acid (Aib), 3-aminoisobutyric acid (βAib), 2-aminopimelic acid, 2,4-diaminobutyric acid (Dbu), desmosine (Des), 2,2'-diaminopimelic acid (Dpm), 2,3-diaminopropionic acid (Dpr), N-ethylglycine (EtGly), N-ethylasparagine (EtAsn), hydroxylysine (Hyl), isohydroxylysine (aHyl), 3-hydroxyproline (3Hyp), 4-hydroxyproline (4Hyp), isodesmosine (Ide), iso-isoleucine (alle), N-methylglycine (MeGly), N-methylisoleucine (Melle), 6-N-methyllysine (MeLys), N-methylvaline (MeVal), norvaline (Nva), norleucine (Nle) and Ornithine (orn). Certainly, all modified α-amino acids may be substituted by corresponding β-, γ- or ω-aminocarboxylic acids.

The term "amino acid" refers to a molecule containing amino and carboxyl. Suitable amino acids comprise, but are not limited to, D- and L-isomers of naturally occurring amino acids, as well as non-naturally occurring amino acids prepared by organic synthesis or other metabolic pathways. As used herein, the term "amino acid" comprises, but is not limited to, an α-amino acid, a natural amino acid, an unnatural amino acid and an amino acid analog.

The term "naturally occurring amino acid" refers to any one of 20 L-amino acids commonly found in peptides that

are synthesized in nature, that is an L-isomer of alanine (Ala or A), arginine (Arg or R), asparagine (Asn or N), aspartic acid (Asp or D), cysteine (Cys or C), glutamic acid (Glu or E), glutamine (Glu or Q), glycine (Gly or G), histidine (His or H), isoleucine (Ile or I), leucine (Leu or 1), lysine (Lys or K), methionine (Met or M), phenylalanine (Phe or F), proline (Pro or P), serine (Ser or S), threonine (Thr or T), tryptophan (Trp or W), tyrosine (Tyr or Y) or valine (Val or V).

The "conserved amino acid substitution" refers to an amino acid substitution in which an amino acid residue is substituted by an amino acid residue having a similar side chain. Families of amino acid residues having similar side chains have been defined in the art. These families comprise amino acids having basic side chains (such as K, R and H), acidic side chains (such as D and E), uncharged polar side chains (such as G, N, Q, S, T, Y and C), nonpolar side chains (such as A, V, L, I, P, F, M and W), β-branched side chains (such as T, V and I) and aromatic side chains (such as Y, F, W and H). Therefore, for example, it is predicted that a non-essential amino acid residue in a polypeptide is preferably substituted by another amino acid residue from a family of the same side chain. Other examples of acceptable substitutions are substitutions based on isosteric considerations (such as a substitution of methionine by norleucine) or other properties (such as a substitution of phenylalanine by 2-thienylalanine).

The polypeptide of the present invention may be prepared by methods well known to those skilled in the art, comprising well-known chemical synthesis methods. Therefore, when the polypeptide or the derivative thereof contains one or more non-standard amino acids, it is very likely to be prepared by chemical synthesis. In addition to preparing by chemical synthesis, the polypeptide or the derivative thereof may also be prepared by coding nucleic acid expression, which is especially suitable for preparing the polypeptide containing only natural amino acids or the derivative thereof, and in this case, well-known methods for preparing a nucleic acid-coded polypeptide sequence may be used (referring to Sambrook et al., Molecular Cloning: A Laboratory Manual, Third Ed., Cold Spring Harbor Laboratory, New York (2001); Ausubel et al., Current Protocols in Molecular Biology, John Wiley and Sons, Baltimore, MD (1999)). The polypeptide may be expressed in organisms and purified by well-known purification technologies.

The term "alkylene" refers to divalent alkyl (that is, -R-).

The term "alkenyl" refers to a linear or branched hydrocarbon chain having one or more carbon-carbon double bonds. The alkenyl moiety contains a specified number of carbon atoms. For example, $C_2$-$C_{10}$ represents that the group contains 2-10 (inclusive) carbon atoms.

The term "alkenyl" refers to a linear or branched hydrocarbon chain having one or more carbon-carbon triple bonds.

As for sequence identity, according to methods known in the art, the sequence identity is calculated by sequence alignment. In order to determine a percentage of identity of two amino acid sequences, the sequences are aligned for optimal comparison. For example, a gap may be introduced into a sequence of a first amino acid sequence for optimal alignment with a second amino acid sequence, and then, amino acid residues in corresponding amino acid positions are compared. When a position in the first sequence is occupied by the same amino acid residue in the corresponding position in the second sequence, the molecule is the same in this position. The percentage identity between two sequences is a function of a number of identical positions shared by the sequences. Therefore, % identity = number of identical positions/total number of overlapping positions x 100.

In this comparison, the sequences may have same length or different lengths. The optimal sequence alignment for determining a comparison window may be carried out by a local homology algorithm of Smith and Waterman (J. Theor. Biol., 1981), a homology alignment algorithm of Needleman and Wunsch (J. Mol. Biol, 1972), and a method for searching similarity of Pearson and Lipman (Proc. Natl. Acad. Sci. U.S.A., 1988). By the computerized implementation of these algorithms (GAP, BESTFIT, FASTA and TFASTA in Wisconsin Genetic Computer Group 7.0, Genetic Computer Group, 575, Science Drive, Madison, Wisconsin) or, for example, publicly available computer software such as BLAST, when the software is used, default parameters are preferably used, such as a gap penalty or an extension penalty. The optimal alignment produced by various methods (that is, the highest percentage of identity produced within a whole range of comparison window) is selected.

In specific embodiments, an amino acid sequence of a stapled peptide has at least 90%, 93% or 95% sequence identity with SEQ ID NO: 1.

The term "pharmaceutical composition" refers to a pharmaceutical composition comprising a therapeutically effective amount of the polypeptide of the present invention and a pharmaceutically acceptable carrier or excipient. As used in the present invention, the "pharmaceutically acceptable carrier" or the "pharmaceutically acceptable excipient" comprises any and all solvents, dispersion media, coatings, antibacterial and antifungal agents, isotonic and absorption delay agents, and physiologically compatible analogues. Examples of the pharmaceutically acceptable carrier or excipient comprise one or more of: water, saline, phosphate buffered saline, glucose, glycerol, ethanol and analogues, and a combination thereof. In any case, the combination preferably comprises an isotonic agent, comprising sugar and a polyol, such as mannitol, sorbitol or sodium chloride; may also comprise a pharmaceutically acceptable substance, comprising a wetting amount or trace amount of auxiliary substance, such as a wetting or emulsifying agent, a preservative or a buffer for improving the storage time and effectiveness of an antibody or antibody part; and may optionally comprise a disintegrant, comprising cross-linked polyvinylpyrrolidone, agar, an alginic acid or a salt thereof, such as sodium alginate. In addition to

the excipient, the pharmaceutical composition may also comprise one or more of: a carrier protein such as serum albumin, a buffer, a binder, a sweetener and other flavoring agents, such as a colorant and polyethylene glycol.

The composition may have many forms, comprising liquid, semi-solid and solid dosage forms, such as a liquid solution (comprising an injectable solution and an infusible solution), a dispersion or a suspension, a tablet, a pill, a powder, a liposome and a suppository. The preferred form will depend on an established route of administration and a therapeutic application. In one embodiment, the composition is in the form of injectable or infusible liquid, such as those similar forms used in passive immunization of human with the antibody. In one embodiment, parenteral (e.g., intravenous, subcutaneous, intraperitoneal and intramuscular) administration is adopted, and in one embodiment, the polypeptide is administered by intravenous injection or infusion. In another embodiment, the polypeptide is administered by intramuscular or subcutaneous injection.

Other administration routes suitable for the pharmaceutical composition comprise, but are not limited to, rectal, transdermal, vaginal, transmucosal or enteral administration.

The "pharmaceutically acceptable salt" of the present invention, that is, a medicinal salt, may be synthesized from the polypeptide and an alkaline or acidic moiety by conventional chemical methods. Generally, such salts may be prepared by allowing free acid forms of these polypeptides to react with stoichiometric amounts of suitable bases (such as a hydroxide, a carbonate, a bicarbonate and the like of Na, Ca, Mg or K), or by allowing free base forms of these polypeptides to react with stoichiometric amounts of suitable acids. Such reactions are usually carried out in water or an organic solvent, or their mixture. Generally, in appropriate circumstances, it is necessary to use a non-aqueous medium such as ether, ethyl acetate, ethanol, isopropanol or acetonitrile. For another example, a list of other suitable salts may be found in "Remington's Pharmaceutical Sciences", 20th version, Mack Publishing Company, Easton, Pa., (1985); and "Handbook of Pharmaceutical Salts: Properties, Selection, and Use", Stahl and Wermuth (Wiley-VCH, Weinheim, Germany, 2002).

The medicinal salt may be a medicinal acid-addition salt, which may be formed by an interaction of the polypeptide of the present invention with an inorganic acid and/or organic acid, for example, a salt formed by the interaction with the inorganic acid, such as a hydrochloric acid, a hydrobromic acid, a nitric acid, a phosphoric acid or a sulfuric acid; and a salt formed by the interaction with the organic acid, such as an acetic acid, a trifluoroacetic acid, a propionic acid, a malonic acid, an oxalic acid, a maleic acid, a fumaric acid, a malic acid, a citric acid, a gluconic acid, a mandelic acid, a tartaric acid, a stearic acid, a succinic acid, a sulfosalicylic acid, a lactic acid, a benzoic acid, a benzenesulfonic acid, a methanesulfonic acid, an ethanesulfonic acid, a toluenesulfonic acid or a naphthalenedisulfonic acid.

The medicinal salt may be a medicinal base-addition salt, which may be formed by an interaction of the polypeptide of the present invention with an inorganic base and/or an organic base. Inorganic bases of salts derived from them comprise, for example, an ammonium salt and metals from Groups I to XII of the Periodic Table. In some embodiments, the salts are derived from sodium, potassium, ammonium, calcium, magnesium, iron, silver, zinc and copper; and particularly suitable salts comprise ammonium, potassium, sodium, calcium and magnesium salts. Organic bases of salts derived from them comprise a primary amine, a secondary amine and a tertiary amine, and substituted amines comprise a naturally occurring substituted amine, a cyclic amine, a basic ion exchange resin and the like. Some organic amines comprise, for example, isopropylamine, benzathine, cholinate, diethanolamine, diethylamine, lysine, meglumine, piperazine and tromethamine.

In addition, the polypeptide disclosed in the present invention and the salt comprising the polypeptide may also exist in the form of their hydrates or in the form of their solvents (such as ethanol and DMSO), and may be used for crystallization. The compound disclosed in the present invention may form a solvate with the pharmaceutically acceptable solvent (comprising water) inherently or by design; and therefore, the compound of the present invention comprises both solvated and unsolvated forms.

## Embodiment

A linear chain precursor of a polypeptide compound and a derivative thereof provided by the present disclosure is synthesized by solid-phase synthesis, and an intramolecular disulfide bond is formed under the catalysis of 2,2'-dithiodipyridine. A synthetic carrier is Fmoc-Cys(Trt)-2-Chlotrityl Resin. In the synthesis process, the Fmoc-Cys(Trt)-2-Chlotrityl Resin is fully swollen in N,N-dimethylformamide (DMF) first, then the solid carrier and an activated amino acid derivative are repeatedly condensed → washed → subjected to Fmoc deprotection → washed → subjected to next round of amino acid condensation to reach a length of a polypeptide chain to be synthesized, and finally, a mixed solution of trifluoroacetic acid: water: triisopropylsilane:
thioanisole (90: 2.5: 2.5: 5, v: v: v: v) reacts with the resin to lyse the polypeptide from the solid carrier, and then precipitated by frozen methyl tert-butyl ether to obtain a solid crude product of the linear chain precursor. The crude product of the linear chain precursor after cutting is subjected to disulfide bond oxidization in an alkaline solution to obtain a target crude product of the polypeptide. The crude product of the polypeptide is purified and separated by a C-18 reversed-phase preparative chromatographic column in an acetonitrile/water system of 0.1% trifluoroacetic acid to obtain a pure product of the

polypeptide and the derivative thereof.

**Experimental reagents**

Fmoc-Cys(Trt)-2-Chlotrityl Resin, Fmoc-Gly-OH, Fmoc-Phe-OH, Fmoc-Gln(Trt)-OH, Fmoc-Cys(Trt)-OH, Fmoc-Ile-OH, Fmoc-Val-OH, Fmoc-Lys(Boc)-OH, Fmoc-Trp(Boc)-OH, Fmoc-Leu-OH, Fmoc-Arg(Pbf)-OH, Fmoc-Thr(tBu)-OH, Fmoc-Tyr(tBu)-OH, Rink Amide-AM Resin, Fmoc-Asn(Trt)-OH and Fmoc-Ser(tBu)-OH are all purchased from GL Biochem, 6-chlorobenzotriazole-1,1,3,3-hexafluorophosphate tetramethylurea (HCTU), trifluoroacetic acid (TFA), 2,2'-dithiodipyridine, ammonium bicarbonate, dimethyl sulfoxide (DMSO) and guanidine hydrochloride are purchased from Aladdin, N,N-dimethylformamide (DMF) is purchased from Energy, dichloromethane (DCM) and 4-methylpiperidine are purchased from Macklin, triisopropylsilane, methyl tert-butyl ether, 4-methylmorpholine (NMM) and thioanisole are purchased from TCL, acetonitrile is purchased from Sigma-Aldrich, and 10×PBS is purchased from Solarbio, and other reagents not specified are all commercially available.

**Example 1. Preparation of compound 30 (corresponding to SEQ ID No. 23)**

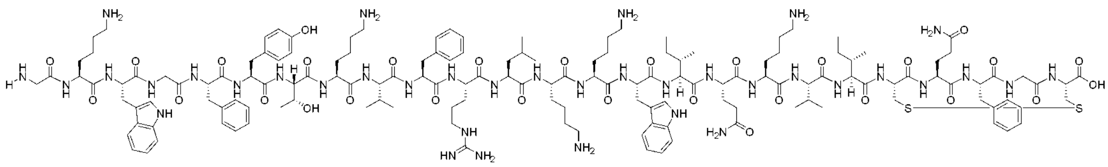

Step 1: synthesis of linear chain precursor peptide chain (G-K-W-G-F-Y-T-K-V-F-R-L-K-K-W-I-Q-K-V-I-C-Q-F-G-C)

226 mg of (0.1 mmol) Fmoc-Cys(Trt)-2-Chlotrityl Resin was fully swollen in DMF for 1 hour. Then, a sequence of the linear chain precursor was synthesized from a second position G of a carboxyl terminal to an amino terminal. In each coupling period, 20% piperidine/DMF (20%v/v, 10 mL) was subjected to Fmoc deprotection twice, for 8 minutes each time. The resin was washed with DMF for 6-8 times until neutral pH was reached. 0.5 mmol Fmoc-AA, 0.5 mmol 6-chlorobenzotriazole-1,1,3,3-hexafluorophosphate tetramethylurea (HCTU) and 1 mmol 4-methylmorpholine (NMM) were dissolved in DMF, and added with the resin to react at room temperature for 1 hour. Before coupling the next amino acid, the resin was washed with DMF for 4-6 times to obtain the linear chain precursor peptide chain. After the linear chain precursor polypeptide was synthesized, the resin was washed with DMF for 5 times and washed with DCM for 5 times. Subsequently, the resin was sucked dry in vacuum.

Step 2: cutting of linear chain precursor peptide chain

A freshly prepared cleavage cocktail (10 mL) trifluoroacetic acid: water: triisopropylsilane: thioanisole (90: 2.5: 2.5: 5, v: v: v: v) was added into the resin obtained in the step 1 to react at room temperature for 2 hours. After the reaction was ended, the reaction solution was filtered, and the resin was washed with trifluoroacetic acid, combined with the reaction solution, and precipitated with 4 times the volume of MTBE to obtain a crude product. The crude product was washed with MTBE for 3 times, and sucked dry in vacuum.

Step 3: formation of intramolecular disulfide bond

The crude product obtained in the step 2 was dissolved in 50% ACN aqueous solution to reach a concentration of 4 mg/mL. 0.5 times equivalent of 2,2'-dithiodipyridine was added with 50 mM ammonium bicarbonate buffer (pH=8.0, containing 50% acetonitrile) to completely dissolve the 2,2'-dithiodipyridine. Finally, a polypeptide solution was slowly dropwise added into the 2,2'-dithiodipyridine solution to reach a final concentration of 2 mg/ml, and shaken at room temperature for 16 hours. Reaction results were monitored by LC-MS. After the reaction was ended, 0.5% (v: v) trifluoroacetic acid was added for quenching.

Step 4: purification and preparation of polypeptide

The mixture was filtered by 0.45 μm membrane and then separated by a reversed-phase high-performance liquid chromatographic system, and buffers were A (0.1% trifluoroacetic acid, aqueous solution) and B (0.1% trifluoroacetic acid,

acetonitrile), wherein a chromatographic column was a BR C-18 (Sepax) reversed-phase chromatographic column, and in a purification process, a detection wavelength of a chromatographic instrument was set to be 230 nm, a flow rate was 15 mL/min, and a gradient was 15-30% acetonitrile, and the operation lasted for 40 minutes. Related fractions of the product were collected and subjected to purity identification by HPLC, and then fractions with purity >95% were combined and freeze-dried to obtain a pure product of the polypeptide.

Step 5: detection and characterization methods

A purity and an intramolecular disulfide bond for compound formation of the pure product of the polypeptide in the step 4 were determined by a combination of analytical high-performance liquid chromatography and liquid chromatography/mass spectrometry, as shown in FIG. 2 and FIG. 1.

The inventor made a series of modifications on the polypeptide compound 30 (comprising the addition or substitution of one or more amino acids), modified polypeptide compounds 1-109 were obtained, and specific sequences and structures of these polypeptides and polypeptide derivatives could be obtained. Similar synthesis and purification as in Example 1 were carried out by Hunan Zonsen PepLib Biotech Inc., and the purity was >95%.

**Example 2. Preparation of compound 105 (corresponding to SEQ ID No. 95)**

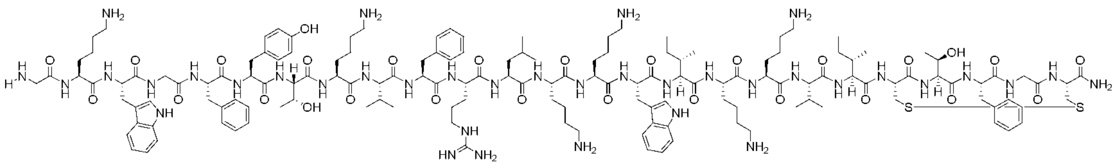

Step 1: synthesis of linear chain precursor peptide chain

Synthesis of linear chain precursor peptide chain of compound 105
G-K-W-G-F-Y-T-K-V-F-R-L-K-K-W-I-K-K-V-I-C-T-F-G-C

294 mg of (0.2 mmol) Rink Amide-AM Resin was fully swollen in DMF for 1 hour. Then, a sequence of the linear chain precursor was synthesized from a first position C of a carboxyl terminal to an amino terminal. In each coupling period, 20% piperidine/DMF (20%v/v, 10 mL) was subjected to Fmoc deprotection twice, for 8 minutes each time. The resin was washed with DMF for 6-8 times until neutral pH was reached. 1.0 mmol Fmoc-AA, 1.0 mmol 6-chlorobenzotriazole-1,1,3,3-hexafluorophosphate tetramethylurea (HCTU) and 2 mmol 4-methylmorpholine (NMM) were dissolved in DMF, and added with the resin to react at room temperature for 1 hour. Before coupling the next amino acid, the resin was washed with DMF for 4-6 times. After the linear chain polypeptide was synthesized, the resin was washed with DMF for 5 times and washed with DCM for 5 times. The resin was sucked dry in vacuum.

Step 2: cutting of linear chain precursor peptide chain

A freshly prepared cleavage cocktail (10 mL) trifluoroacetic acid: water: triisopropylsilane: thioanisole (90: 2.5: 2.5: 5, v: v: v: v) was added into the resin obtained in the step 1 to react at room temperature for 2 hours. After the reaction was ended, the reaction solution was filtered, and the resin was washed with trifluoroacetic acid, combined with the reaction solution, and precipitated with 4 times the volume of cold MTBE to obtain a crude product. The crude product was washed with MTBE for 3 times, and sucked dry in vacuum.

Step 3: formation of intramolecular disulfide bond

The crude product obtained in the step 2 was added with DMSO to be fully dissolved (a volume of the DMSO was 20% of a total volume of the reaction system). A phosphate\guanidine hydrochloride buffer (pH=7.0, containing 50% ACN) was prepared. 1 x PBS: 6 M guanidine hydrochloride (80: 20, v: v) buffer was mixed with acetonitrile according to 1: 1 to obtain the phosphate\guanidine hydrochloride buffer (pH=7.0, containing 50% ACN). Then, a polypeptide solution was slowly dropwise added into the above buffer to reach a final concentration of 1 mg/ml, and shaken at room temperature for 16 hours. Reaction results were monitored by LC-MS. After the reaction was ended, the product was directly purified and prepared.

Step 4: purification and preparation of polypeptide

The mixture was filtered by 0.45 μm membrane and then separated by a reversed-phase high-performance liquid chromatographic system, and buffers were A (0.1% trifluoroacetic acid, aqueous solution) and B (0.1% trifluoroacetic acid, acetonitrile), wherein a chromatographic column was a BR C-18 (Sepax) reversed-phase chromatographic column, and in a purification process, a detection wavelength of a chromatographic instrument was set to be 230 nm, a flow rate was 15 mL/min, and a gradient was 25-45% acetonitrile, and the operation lasted for 40 minutes. Related fractions of the product were collected and subjected to purity identification by HPLC, and then fractions with purity >95% were combined and freeze-dried to obtain a pure product of the polypeptide.

Step 5: detection and characterization methods

A purity and an intramolecular disulfide bond for compound formation of the pure product of the polypeptide in the step 4 were determined by a combination of analytical high-performance liquid chromatography and liquid chromatography/mass spectrometry, as shown in FIG. 3 and FIG. 4.

**Example 3. Preparation of compound 106 (corresponding to SEQ ID No. 96)**

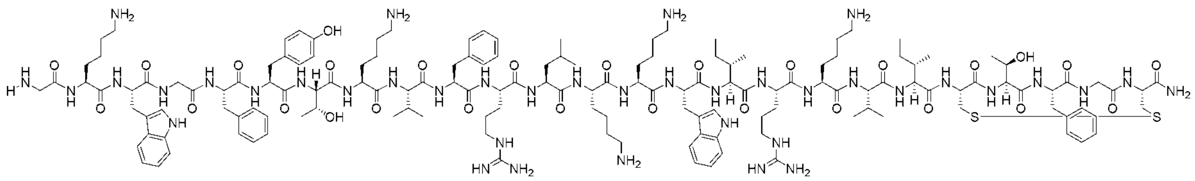

Step 1: synthesis of linear chain precursor peptide chain

Synthesis of linear chain precursor peptide chain of compound 106
G-K-W-G-F-Y-T-K-V-F-R-L-K-K-W-I-R-K-V-I-C-T-F-G-C

294 mg of (0.2 mmol) Rink Amide-AM Resin was fully swollen in DMF for 1 hour. Then, a sequence of the linear chain precursor was synthesized from a first position C of a carboxyl terminal to an amino terminal. In each coupling period, 20% piperidine/DMF (20%v/v, 10 mL) was subjected to Fmoc deprotection twice, for 8 minutes each time. The resin was washed with DMF for 6-8 times until neutral pH was reached. 1.0 mmol Fmoc-AA, 1.0 mmol 6-chlorobenzotriazole-1,1,3,3-hexafluorophosphate tetramethylurea (HCTU) and 2 mmol 4-methylmorpholine (NMM) were dissolved in DMF, and added with the resin to react at room temperature for 1 hour. Before coupling the next amino acid, the resin was washed with DMF for 4-6 times. After the linear chain polypeptide was synthesized, the resin was washed with DMF for 5 times and washed with DCM for 5 times. The resin was sucked dry in vacuum.

Step 2: cutting of linear chain precursor peptide chain

A freshly prepared cleavage cocktail (10 mL) trifluoroacetic acid: water: triisopropylsilane: thioanisole (90: 2.5: 2.5: 5, v: v: v: v) was added into the resin obtained in the step 1 to react at room temperature for 2 hours. After the reaction was ended, the reaction solution was filtered, and the resin was washed with trifluoroacetic acid, combined with the reaction solution, and precipitated with 4 times the volume of cold MTBE to obtain a crude product. The crude product was washed with MTBE for 3 times, and sucked dry in vacuum.

Step 3: formation of intramolecular disulfide bond

The crude product obtained in the step 2 was added with DMSO to be fully dissolved (a volume of the DMSO was 20% of a total volume of the reaction system). A phosphate\guanidine hydrochloride buffer (pH=7.0, containing 50% ACN) was prepared. 1 x PBS: 6 M guanidine hydrochloride (80: 20, v: v) buffer was mixed with acetonitrile according to 1: 1 to obtain the phosphate\guanidine hydrochloride buffer (pH=7.0, containing 50% ACN). Then, a polypeptide solution was slowly dropwise added into the above buffer to reach a final concentration of 1 mg/ml, and shaken at room temperature for 16 hours. Reaction results were monitored by LC-MS. After the reaction was ended, the product was directly purified and prepared.

Step 4: purification and preparation of polypeptide

The mixture was filtered by 0.45 μm membrane and then separated by a reversed-phase high-performance liquid chromatographic system, and buffers were A (0.1% trifluoroacetic acid, aqueous solution) and B (0.1% trifluoroacetic acid, acetonitrile), wherein a chromatographic column was a BR C-18 (Sepax) reversed-phase chromatographic column, and in a purification process, a detection wavelength of a chromatographic instrument was set to be 230 nm, a flow rate was 15 mL/min, and a gradient was 25-40% acetonitrile, and the operation lasted for 40 minutes. Related fractions of the product were collected and subjected to purity identification by HPLC, and then fractions with purity >95% were combined and freeze-dried to obtain a pure product of the polypeptide.

Step 5: detection and characterization methods

A purity and an intramolecular disulfide bond for compound formation of the pure product of the polypeptide in the step 4 were determined by a combination of analytical high-performance liquid chromatography and liquid chromatography/mass spectrometry, as shown in FIG. 5 and FIG. 6.

**Example 4. Preparation of compound 108 (corresponding to SEQ ID No. 98)**

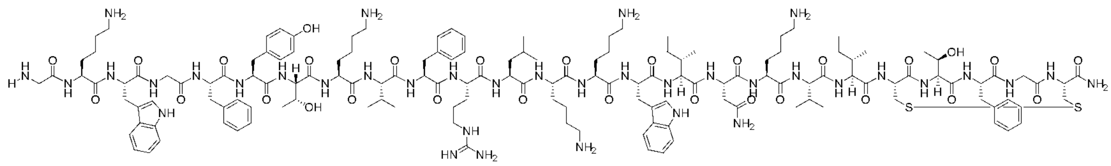

Step 1: synthesis of linear chain precursor peptide chain

Synthesis of linear chain precursor peptide chain of compound 108
G-K-W-G-F-Y-T-K-V-F-R-L-K-K-W-I-N-K-V-I-C-T-F-G-C

294 mg of (0.2 mmol) Rink Amide-AM Resin was fully swollen in DMF for 1 hour. Then, a sequence of the linear chain precursor was synthesized from a first position C of a carboxyl terminal to an amino terminal. In each coupling period, 20% piperidine/DMF (20%v/v, 10 mL) was subjected to Fmoc deprotection twice, for 8 minutes each time. The resin was washed with DMF for 6-8 times until neutral pH was reached. 1.0 mmol Fmoc-AA, 1.0 mmol 6-chlorobenzotriazole-1,1,3,3-hexafluorophosphate tetramethylurea (HCTU) and 2 mmol 4-methylmorpholine (NMM) were dissolved in DMF, and added with the resin to react at room temperature for 1 hour. Before coupling the next amino acid, the resin was washed with DMF for 4-6 times. After the linear chain polypeptide was synthesized, the resin was washed with DMF for 5 times and washed with DCM for 5 times. The resin was sucked dry in vacuum.

Step 2: cutting of linear chain precursor peptide chain

A freshly prepared cleavage cocktail (10 mL) trifluoroacetic acid: water: triisopropylsilane: thioanisole (90: 2.5: 2.5: 5, v: v: v: v) was added into the resin obtained in the step 1 to react at room temperature for 2 hours. After the reaction was ended, the reaction solution was filtered, and the resin was washed with trifluoroacetic acid, combined with the reaction solution, and precipitated with 4 times the volume of cold MTBE to obtain a crude product. The crude product was washed with MTBE for 3 times, and sucked dry in vacuum.

Step 3: formation of intramolecular disulfide bond

The crude product obtained in the step 2 was added with DMSO to be fully dissolved (a volume of the DMSO was 20% of a total volume of the reaction system). A phosphate\guanidine hydrochloride buffer (pH=7.0, containing 50% ACN) was prepared. 1 x PBS: 6 M guanidine hydrochloride (80: 20, v: v) buffer was mixed with acetonitrile according to 1: 1 to obtain the phosphate\guanidine hydrochloride buffer (pH=7.0, containing 50% ACN). Then, a polypeptide solution was slowly dropwise added into the above buffer to reach a final concentration of 1 mg/ml, and shaken at room temperature for 16 hours. Reaction results were monitored by LC-MS. After the reaction was ended, the product was directly purified and prepared.

Step 4: purification and preparation of polypeptide

The mixture was filtered by 0.45 μm membrane and then separated by a reversed-phase high-performance liquid chromatographic system, and buffers were A (0.1% trifluoroacetic acid, aqueous solution) and B (0.1% trifluoroacetic acid, acetonitrile), wherein a chromatographic column was a BR C-18 (Sepax) reversed-phase chromatographic column, and in a purification process, a detection wavelength of a chromatographic instrument was set to be 230 nm, a flow rate was 15 mL/min, and a gradient was 30-50% acetonitrile, and the operation lasted for 40 minutes. Related fractions of the product were collected and subjected to purity identification by HPLC, and then fractions with purity >95% were combined and freeze-dried to obtain a pure product of the polypeptide.

Step 5: detection and characterization methods

A purity and an intramolecular disulfide bond for compound formation of the pure product of the polypeptide in the step 4 were determined by a combination of analytical high-performance liquid chromatography and liquid chromatography/mass spectrometry, as shown in FIG. 7 and FIG. 8.

**Example 5. Preparation of compound 109 (corresponding to SEQ ID No. 98)**

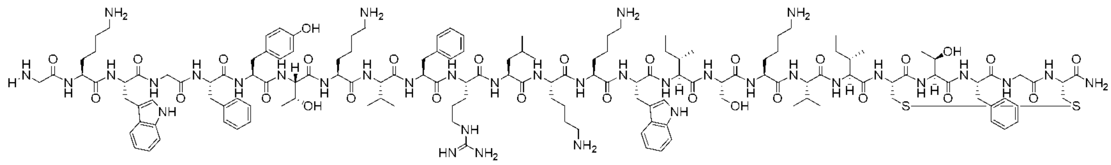

Step 1: synthesis of linear chain precursor peptide chain

Synthesis of linear chain precursor peptide chain of compound 109
G-K-W-G-F-Y-T-K-V-F-R-L-K-K-W-I-S-K-V-I-C-T-F-G-C

294 mg of (0.2 mmol) Rink Amide-AM Resin was fully swollen in DMF for 1 hour. Then, a sequence of the linear chain precursor was synthesized from a first position C of a carboxyl terminal to an amino terminal. In each coupling period, 20% piperidine/DMF (20%v/v, 10 mL) was subjected to Fmoc deprotection twice, for 8 minutes each time. The resin was washed with DMF for 6-8 times until neutral pH was reached. 1.0 mmol Fmoc-AA, 1.0 mmol 6-chlorobenzotriazole-1,1,3,3-hexafluorophosphate tetramethylurea (HCTU) and 2 mmol 4-methylmorpholine (NMM) were dissolved in DMF, and added with the resin to react at room temperature for 1 hour. Before coupling the next amino acid, the resin was washed with DMF for 4-6 times. After the linear chain polypeptide was synthesized, the resin was washed with DMF for 5 times and washed with DCM for 5 times. The resin was sucked dry in vacuum.

Step 2: cutting of linear chain precursor peptide chain

A freshly prepared cleavage cocktail (10 mL) trifluoroacetic acid: water: triisopropylsilane: thioanisole (90: 2.5: 2.5: 5, v: v: v: v) was added into the resin obtained in the step 1 to react at room temperature for 2 hours. After the reaction was ended, the reaction solution was filtered, and the resin was washed with trifluoroacetic acid, combined with the reaction solution, and precipitated with 4 times the volume of cold MTBE to obtain a crude product. The crude product was washed with MTBE for 3 times, and sucked dry in vacuum.

Step 3: formation of intramolecular disulfide bond

The crude product obtained in the step 2 was added with DMSO to be fully dissolved (a volume of the DMSO was 20% of a total volume of the reaction system). A phosphate\guanidine hydrochloride buffer (pH=7.0, containing 50% ACN) was prepared. 1 x PBS: 6 M guanidine hydrochloride (80: 20, v: v) buffer was mixed with acetonitrile according to 1: 1 to obtain the phosphate\guanidine hydrochloride buffer (pH=7.0, containing 50% ACN). Then, a polypeptide solution was slowly dropwise added into the above buffer to reach a final concentration of 1 mg/ml, and shaken at room temperature for 16 hours. Reaction results were monitored by LC-MS. After the reaction was ended, the product was directly purified and prepared.

Step 4: purification and preparation of polypeptide

The mixture was filtered by 0.45 μm membrane and then separated by a reversed-phase high-performance liquid chromatographic system, and buffers were A (0.1% trifluoroacetic acid, aqueous solution) and B (0.1% trifluoroacetic acid, acetonitrile), wherein a chromatographic column was a BR C-18 (Sepax) reversed-phase chromatographic column, and in a purification process, a detection wavelength of a chromatographic instrument was set to be 230 nm, a flow rate was 15 mL/min, and a gradient was 25-40% acetonitrile, and the operation lasted for 40 minutes. Related fractions of the product were collected and subjected to purity identification by HPLC, and then fractions with purity >95% were combined and freeze-dried to obtain a pure product of the polypeptide.

Step 5: detection and characterization methods

A purity and an intramolecular disulfide bond for compound formation of the pure product of the polypeptide in the step 4 were determined by a combination of analytical high-performance liquid chromatography and liquid chromatography/mass spectrometry, as shown in FIG. 9 and FIG. 10.

**Biological evaluation**

**Example 6** Functional test of minimal inhibitory concentrations (MIC) of molecules

According to the present invention, antibacterial activities of compounds were determined by measuring the minimal inhibitory concentrations (MIC) of the molecules to Escherichia coli ATCC 25922, Staphylococcus aureus ATCC 29213 and Pseudomonas aeruginosa ATCC 27853.

Bacteria used for testing the minimal inhibitory concentrations were frozen in a refrigerator at -80°C, and thawed 2 days in advance when used. A little of frozen liquid bacteria were scraped with a sterile inoculating loop, subjected to streak inoculation on a TSA solid medium dish, and cultured an ordinary incubator at 35±2°C for about 20 hours. 5-10 bacterial colonies with similar morphology were selected from the above-mentioned dish with the sterile inoculating loop, subjected to streak inoculation again on a corresponding solid medium dish, and then cultured in an ordinary incubator at 35±2°C for about 20 hours. 5-10 bacterial colonies were selected from a solid culture dish and resuspended in 500 μl of sterile saline (0.9% NaCl), and then OD 600 was adjusted to be ~0.15 with a spectrophotometer. The bacteria were diluted 300 times with 1.02 x CAMHB (containing 0.02% BSA) balanced to room temperature to reach an inoculation concentration of ~2 x $10^5$ CFU/ml.

An alternative polypeptide was dissolved into 3.2 mg/ml standby solution with DMSO, and the standby solution was gradiently diluted with DMSO twice in sequence, with 11 dilutions. 2 μl of polypeptide compound was transferred to a corresponding well of a test plate, and more than 98 μl of prepared bacterial inoculum was added into the test plate. A maximal detection concentration of the compound was 256 μg/ml. The test plate was centrifuged in a centrifuge at 800 rpm for 30 seconds, and then vibrated on a vibrator at 400 rpm for 1 minute to be mixed evenly, and then, the mixture was cultured in an ordinary incubator at 35±2°C for 20 hours. The test plate was placed on a plate reading instrument, and a reflector was adjusted to observe and record the growth of the bacteria in each well. Meanwhile, each test plate was photographed by a QCount system, and an OD600 value of the bacteria in each well was read by SpectraMax plus 384. Test results were as shown in Table 1 (serial numbers in Table 1 represented serial numbers of the compounds, which were consistent with those in the previous text):

Table 1: Minimal inhibitory concentrations (MIC) of compounds

| MIC (μg/ML) | | | | | | | |
|---|---|---|---|---|---|---|---|
| **Serial No.** | **Escherichia coli ATCC 25922** | **Staphylococcus aureus ATCC 29213** | **Pseudomonas aeruginosa ATCC 27853** | **Serial No.** | **Escherichia coli ATCC 25922** | **Staphylococcu s aureus ATCC 29213** | **Pseudomonas aeruginosa ATCC 27853** |
| **1** | 32 | 32 - 64 | N.D. | **54** | 16 - 32 | 8 | N.D. |
| **9** | 8 - 16 | 16 - 32 | N.D. | **55** | 16 - 32 | 16 | N.D. |
| **10** | 8 | 8 | N.D. | **56** | 32 | 32 | N.D. |
| **11** | 16 | 16 | N.D. | **58** | 16 | 16 | 128 |
| **12** | 4 - 8 | 4 | N.D. | **59** | 8 - 16 | 16 | 128 |
| **13** | 32 | 64 | N.D. | **60** | 16 | 16 | 128 |
| **14** | 16 | 16 | N.D. | **62** | 32 | 32 - 16 | 256 |
| **16** | 4 - 8 | 16 | N.D. | **63** | 32 | 32 - 16 | 256 |
| **17** | 16 - 32 | 32 | N.D. | **64** | 32 | 32 | 128 |
| **19** | 8 - 16 | 16 | N.D. | **65** | 64 | 64 - 32 | 256 |
| **20** | 8 - 16 | 16 | N.D. | **66** | 64 | 64 - 32 | 256 |
| **21** | 8 - 16 | 16 | N.D. | **74** | 8 - 16 | 8 | 128 |
| **22** | 8 - 16 | 8 | N.D. | **75** | 16 - 32 | 16 | 128 |
| **23** | 8 | 8 | N.D. | **76** | 8 - 16 | 4-8 | 64 |
| **24** | 16 | 16 | N.D. | **77** | 16 | 16 | 256 |
| **25** | 4 | 4 | N.D. | **78** | 16 | 16 | 128 |
| **26** | 8 | 4 | N.D. | **79** | 32 | 16 | 256 |
| **27** | 8 | 4 | N.D. | **80** | 8 - 16 | 4 - 8 | 64 |
| **28** | 16 | 8 | N.D. | **84** | 16 - 32 | 4 | >256 |
| **29** | 8 | 8 | N.D. | **85** | 2 - 4 | 2 | 32 |
| **30** | 2 - 4 | 2 - 4 | 64 | **86** | 16 - 32 | 4 - 8 | >256 |
| **31** | 16 | 16 | N.D. | **87** | 2 - 4 | 2 | 64 |
| **32** | 8 - 16 | 8 | N.D. | **88** | 2 | 8 - 16 | 128 |
| **33** | 32 | 32 | N.D. | **89** | 2 | 4 | 64 |

(continued)

| MIC (μg/ML) | | | | | | | |
|---|---|---|---|---|---|---|---|
| **Serial No.** | **Escherichia coli ATCC 25922** | **Staphylococcus aureus ATCC 29213** | **Pseudomonas aeruginosa ATCC 27853** | **Serial No.** | **Escherichia coli ATCC 25922** | **Staphylococcu s aureus ATCC 29213** | **Pseudomonas aeruginosa ATCC 27853** |
| **34** | 16 | 16 | N.D. | **90** | 2 - 4 | 2 | 64 |
| **35** | 8 - 16 | 8 | N.D. | **91** | 8 | 2 | 32 |
| **36** | 8 | 8 | N.D. | **92** | 8 | 8 | 64 |
| **37** | 8 | 16 | N.D. | **93** | 2 - 4 | 8 | 64 |
| **38** | 8 - 16 | 32 | N.D. | **94** | 2 | 4 | 64 |
| **39** | 8 - 16 | 16 | N.D. | **95** | 2 - 4 | 4 | 64 |
| **40** | 8 - 16 | 16 | N.D. | **96** | 2 | 4 | 32 |
| **41** | 32 | 64 | N.D. | **97** | 4 - 8 | 4 | 32 - 64 |
| **42** | 16 | 16 | N.D. | **98** | 4 - 8 | 8 | 64 |
| **43** | 8 | 8 | N.D. | **99** | 4 - 8 | 8 | 64 |
| **44** | 16 | 8 | N.D. | **100** | 8 | 8 | 64 |
| **45** | 8 | 8 | N.D. | **101** | 8 - 16 | 8 | 64 - 128 |
| **46** | 16 - 32 | 16 | N.D. | **102** | 8 | 4 | 32 - 64 |
| **47** | 16 - 32 | 32 | N.D. | **103** | 8 | 8 | 64 |
| **48** | 32 | 32 - 64 | N.D. | **104** | 4 | 4 | 64 |
| **49** | 16 | 32 | N.D. | **105** | 4 | 4 | 16 - 32 |
| **51** | 32 - 64 | 64 | N.D. | **106** | 2 - 4 | 2 | 16 |
| **52** | 64 | 32 | N.D. | **108** | 2 - 4 | 2 | N.D. |
| **53** | 16 - 32 | 16 | N.D. | **109** | 2 - 4 | 2 - 4 | 32 |

Escherichia coli, Staphylococcus aureus and Pseudomonas aeruginosa were common pathogenic strains of skin wound infection. According to the data in Table 1, the polypeptide compounds provided by the present invention had good bacteriostatic effects on the Escherichia coli ATCC 25922, the Staphylococcus aureus ATCC 29213 and the Pseudomonas aeruginosa ATCC 27853, the minimal inhibitory concentrations (MIC) of the polypeptide compounds to the Escherichia coli ATCC 25922 and the Staphylococcus aureus ATCC 29213 were 2-64 μg/mL, and the minimal inhibitory concentrations (MIC) of the polypeptide compounds to the Pseudomonas aeruginosa ATCC 27853 were 32-256 μg/mL.

**Example 7** Functional test of radial diffusion assay (RDA)

According to the present invention, antibacterial activities of different polypeptides were evaluated by measuring diameters of inhibition zones produced by molecules to the Escherichia coli ATCC 25922, the Staphylococcus aureus ATCC 29213 and the Pseudomonas aeruginosa ATCC 27853.

Bacteria were frozen in a refrigerator at -80°C, and thawed 2 days in advance when used. A little of frozen bacteria were scraped with a sterile inoculating loop, subjected to streak inoculation on a TSA solid medium dish, and cultured an incubator at 35±2°C for about 20 hours. Several bacterial colonies were respectively selected from a solid culture dish and resuspended in 1 mL of TSB, and OD 600 was adjusted to be 1.0 with a spectrophotometer. The resuspended bacteria were re-inoculated into 10 mL TSB at a ratio of 1: 100, and cultured at 37°C and 200 rpm to a mid-log phase. The culture was centrifuged at 4000 rpm for 10 minutes, then a supernatant was discarded, and a precipitate was resuspended in an equal volume of 10 mM Tris buffer (pH=7.4). OD 600 was adjusted to be ~0.15 with a spectrophotometer. A compound to be tested was diluted to be 0.8 mg/mL, and then used for detecting Pseudomonas aeruginosa, Escherichia coli and Staphylococcus aureus respectively. Bottom-layer agar with a TSB concentration of 0.03% (W/V), a low-electroosmotic agarose concentration of 1% (W/V) and a Tween-20 concentration of 0.02% (V/V) was prepared, autoclaved and then cooled to 42~46°C. Bacteria with the OD600 of 0.15 were added into the bottom-layer agar at a ratio of 1: 300 and mixed evenly, and then the mixture was poured into a culture dish and solidified at room temperature. 9 sample injection wells with a diameter of 4 mm were punched in the culture dish with an agar diffusion test punching bear, and 6 μL of the compound to be tested (0.8 mg/mL) was added into the wells according to FIG. 1, and incubated at 37°C for 3 hours to make the sample fully diffuse into the bottom-layer agar. Top-layer agar with a TSB concentration of 6% (W/V) and a low electroosmotic agarose concentration of 1% (W/V) was prepared, autoclaved and then cooled to 42~46°C, and 15 mL of the top-layer agar was added into the above culture dish, fully solidified and then cultured at 37°C for about 20 hours, wherein the agar containing Pseudomonas aeruginosa was invertedly placed on a 15 cm culture dish to make the bottom-layer agar containing the bacteria contact with the air, so that the bacteria could grow fully. The inoculated bacteria solution was diluted from $10^{-1}$ to $10^{-3}$ with a liquid medium in sequence. 100 μl of the above bacterial diluent was evenly coated in a TSA dish. 10 minutes after the medium was absorbed by TSA, the dish was inverted and cultured in an incubator at 35±2°C for 20 hours. Each culture dish was photographed by a QCount system, and a diameter of each inhibition zone was measured and recorded. Results were as shown in Table 2, wherein serial numbers in the table were serial numbers of the compounds, which were consistent with those in the previous text.

Table 2: Radial diffusion assay (RDA) of antibacterial activities of compounds

| Diameter of inhibition zones (mm) | | | | | | | |
|---|---|---|---|---|---|---|---|
| **Serial No.** | **Escherichia coli ATCC 25922** | **Staphylococcus aureus ATCC 29213** | **Pseudomonas aeruginosa ATCC 27853** | **Serial No.** | **Escherichia coli ATCC 25922** | **Staphylococcus aureus ATCC 29213** | **Pseudomonas aeruginosa ATCC 27853** |
| **1** | 8 | 8 | 7 | **54** | 8 | 7 | 7 |
| **9** | 8.5 | 9 | 7 | **55** | 9 | 8.5 | 8 |
| **10** | 9 | 8 | 7.5 | **56** | 7.5 | 6 | 6 |
| **11** | 8 | 7 | 7 | **57** | 6 | 6 | 6 |
| **12** | 9 | 7.5 | 8 | **58** | 7.5 | 6.5 | 8 |
| **13** | 8.5 | 7.5 | 7.5 | **59** | 8 | 7 | 7 |
| **14** | 8 | 8 | 7.5 | **60** | 8 | 7 | 8 |
| **15** | 7.5 | 8 | 7 | **61** | 7 | 6.5 | 8 |
| **16** | 8.5 | 11.5 | 9.5 | **62** | 7 | 6 | 7 |
| **17** | 9.5 | 7 | 6.5 | **63** | 6 | 5.5 | 7.5 |
| **18** | 8.5 | 8 | 8 | **64** | 7 | 6 | 7 |
| **19** | 8 | 9.5 | 8 | **65** | 6 | 5 | 6 |
| **20** | 10 | 8 | 8 | **66** | 8 | 5.5 | 6 |
| **21** | 8.5 | 8 | 8 | **68** | 6.5 | 5.5 | 6.5 |
| **22** | 9 | 6.5 | 6 | **69** | 7 | 5 | 6.5 |
| **23** | 9 | 6.5 | 7 | **70** | 7 | 5 | 6.5 |
| **24** | 8.5 | 6.5 | 6.5 | **71** | 6 | 5 | 6 |
| **25** | 10 | 9 | 8 | **72** | 6.5 | 4 | 5.5 |
| **26** | 10 | 9 | 8 | **73** | 7 | 6 | 6.5 |
| **27** | 8 | 6 | 6.5 | **74** | 8.5 | 7.5 | 8 |
| **28** | 8 | 6.5 | 6 | **75** | 7.5 | 7 | 7 |
| **29** | 8 | 8.5 | 7 | **76** | 9 | 8 | 7.5 |
| **30** | 10 | 9.5 | 9.5 | **77** | 8 | 6 | 7 |
| **31** | 8 | 7.5 | 7 | **78** | 8.5 | 6 | 8 |

(continued)

| Diameter of inhibition zones (mm) | | | | | | | |
|---|---|---|---|---|---|---|---|
| **Serial No.** | **Escherichia coli ATCC 25922** | **Staphylococcus aureus ATCC 29213** | **Pseudomonas aeruginosa ATCC 27853** | **Serial No.** | **Escherichia coli ATCC 25922** | **Staphylococcus aureus ATCC 29213** | **Pseudomonas aeruginosa ATCC 27853** |
| **32** | 9 | 8 | 8 | **79** | 7 | 5 | 7 |
| **33** | 7.5 | 7 | 7 | **80** | 9 | 6 | 8 |
| **34** | 8.5 | 8 | 8 | **84** | 7 | 8 | 7 |
| **35** | 8 | 9 | 7 | **85** | 8 | 9 | 8.5 |
| **36** | 8 | 8.5 | 7.5 | **86** | 7 | 8 | 7.5 |
| **37** | 7.5 | 7 | 6.5 | **87** | 8 | 9 | 7 |
| **38** | 8.5 | 8 | 7.5 | **88** | 7.5 | 11 | 8 |
| **39** | 8 | 8.5 | 7 | **89** | 7.5 | 11 | 9.5 |
| **40** | 8 | 9 | 7.5 | **90** | 9 | 9 | 7 |
| **41** | 8 | 8 | 7 | **91** | 8 | 8.5 | 7 |
| **42** | 7.5 | 8 | 7 | **92** | 8 | 10 | 8 |
| 43 | 8 | 7 | 6.5 | **93** | 8 | 11 | 8 |
| **44** | 9 | 9 | 7.5 | **94** | 8 | 10 | 8 |
| **45** | 9 | 7.5 | 7.5 | **95** | 8 | 10.5 | 8 |
| **46** | 7 | 7 | 6 | **96** | 8 | 8 | 7 |
| **47** | 8.5 | 9.5 | 8 | **98** | 6 | 7 | 6 |
| **48** | 8 | 8 | 7 | **100** | 6 | 6 | 6 |
| **49** | 7 | 9 | 8 | **102** | 6 | 7 | 6 |
| **50** | 7 | 6 | 6 | **105** | 7 | 8 | 7.5 |
| **51** | 8.5 | 7.5 | 7 | **106** | 9 | 8 | 7.5 |
| **52** | 8 | 7 | 7 | **108** | 8 | 8 | 8 |
| **53** | 8 | 7.5 | 7 | **109** | 7 | 7 | 7 |

According to the data in Table 1, the compounds provided by the present invention had good antibacterial effects on the Escherichia coli ATCC 25922, the Staphylococcus aureus ATCC 29213 and the Pseudomonas aeruginosa ATCC 27853, and the molecules still had antibacterial effects after diffusing in middle-layer agar. The diameters of the inhibition zones of the compounds to the Escherichia coli ATCC 25922, the Staphylococcus aureus ATCC 29213 and the Pseudomonas aeruginosa ATCC 27853 were 5-11 mm.

**Example 8** Inhibitory effects (IC50) of polypeptides on expression of fluorescein reporter gene in TLR4 cells activated by LPS

Experimental cells were HEK293/TLR4/NF-kB-Luc cells (Cat: CBP74128) produced by Nanjing Cobioer Biotechnology Co., Ltd., and the cells were thawed and cultured according to cell culture requirements. One-Step Luciferase Assay System (Bioscience Cat: 60690) was selected as a detection kit, and a reagent was prepared and used according to the instruction.

The HEK293/TLR4/NF-kB-Luc cells were cultured and grown in a medium (MEM, 10% FBS, 1% Non-essential amino acids (NEAA), 1 mM Na pyruvate, 100 μg/ml Hygromycin B, 1% Puromycin). When a cell growth density reached 80-90% of a culture bottle, the cells were moistened with DPBS first, and then digested with 0.25% trypsin (containing 0.5 mM EDTA). A cell suspension was collected into a centrifuge tube and centrifuged at 1000 rpm for 3 minutes, and a supernatant medium was removed. 6-8 ml of fresh growth medium was added to resuspend the cells, and the cells were subcultured according to a ratio of 1: 3~1: 8, and cultured in an incubator at 37°C under 5% $CO_2$. After the cells were subcultured, medium change or cell subculture was carried out every 2-3 days.

The HEK293/TLR4/NF-kB-Luc cells were subcultured and expanded to a required number of cells. After the cells were digested and resuspended, a cell suspension was spread in a 384-well white transparent-bottom cell culture plate according to 6000 cells/well, 25 μl/well, and cultured at 37°C under 5% $CO_2$ for 4 days for sample detection.

A use concentration of LPS was determined:

The lipopolysaccharide (LPS) was dissolved with sterilized water to be 5 mg/ml, diluted with 1×Loading buffer to reach a concentration of 60 μg/ml (6×), and gradiently diluted with 1×Loading buffer 3 times in sequence, with a total of 12 concentrations. 5 μl of diluted LPS solution was transferred into the 384-well white transparent-bottom cell culture plate which had been incubated for 4 days, and incubated at 37°C for 6 hours, the Luciferase Assay System reagent was used to dilute a liquid A and a liquid B according to a volume ratio of 100: 1 one hour in advance, the Luciferase Assay System reagent was added into the above cell plate according to 30 μl/well, and the cell plate was cultured in the dark at room temperature, and shaken at 350 rpm for 15 minutes. A chemiluminescence activity was detected by a microplate reader Cytation5, a range value of EC80 of the LPS was 0.06-0.1 μg/ml, and the EC80 concentration was selected for an experiment.

Inhibitory effects of polypeptides on luciferase reporter gene in TLR4 cells activated by LPS:
The polypeptide was dissolved with sterilized water to be 500 μM, diluted with 1xLoading buffer to reach a concentration of 120 μM (12×), and gradiently diluted with 1×Loading buffer 3 times in sequence, with a total of 7 concentrations. The LPS was diluted with 1×Loading buffer to be 1.2 μg/ml (12×) .

25 μl of the gradiently diluted polypeptide (12×) with different concentration and 25 μl of the LPS (12×) were premixed in the same volume, and 5 μl of polypeptide and LPS premix was added into the 384-well white transparent-bottom cell plate which had been incubated for 4 days, and incubated at 37°C for 6 hours. The Luciferase Assay System reagent was used to dilute a liquid A and a liquid B according to a volume ratio of 100: 1 one hour in advance, the Luciferase Assay System reagent was added into the above cell plate according to 30 μl/well, and the cell plate was cultured in the dark at room temperature, and shaken at 350 rpm for 15 minutes. A chemiluminescence activity was detected by the Cytation5. Specific numerical values were as shown in Table 3 (IC50 values of TCP-25 were 6.81±1.4 μm, and serial numbers in the table represented serial numbers of the compounds, which were consistent with those in the previous text).

Table 3: Inhibitory effects (IC50) of compounds on expression of fluorescein reporter gene in TLR4 cells activated by LPS

| Serial No. | Activity multiple relative to TCP-25 | Serial No. | Activity multiple relative to TCP-25 | Serial No. | Activity multiple relative to TCP-25 |
|---|---|---|---|---|---|
| **1** | 1.17 | **45** | 1.04 | **79** | 7.97 |
| **9** | 1.26 | **46** | 1.51 | **80** | 6.77 |
| **10** | 1.60 | **49** | 1.13 | **84** | 1.37 |
| **11** | 2.17 | **55** | 1.20 | **85** | 2.48 |
| **12** | 2.83 | **56** | 1.86 | **86** | 1.69 |

(continued)

| Serial No. | Activity multiple relative to TCP-25 | Serial No. | Activity multiple relative to TCP-25 | Serial No. | Activity multiple relative to TCP-25 |
|---|---|---|---|---|---|
| **13** | 2.00 | **57** | 0.69 | **87** | 2.54 |
| **14** | 1.30 | **58** | 3.62 | **88** | 0.80 |
| **19** | 1.55 | **59** | 2.54 | **89** | 1.84 |
| **21** | 1.38 | **60** | 3.26 | **90** | 3.28 |
| **22** | 1.78 | **61** | 6.12 | **91** | 1.85 |
| **23** | 1.23 | **62** | 4.96 | **92** | 0.90 |
| **24** | 1.51 | **63** | 6.70 | **93** | 0.92 |
| **25** | 1.55 | **64** | 5.83 | **94** | 1.43 |
| **27** | 2.17 | **65** | 9.00 | **95** | 1.70 |
| **28** | 1.34 | **66** | 8.75 | **96** | 3.83 |
| **29** | 1.04 | **67** | 4.47 | **97** | 7.92 |
| **30** | 3.61 | **68** | 2.76 | **98** | 26.19 |
| **32** | 1.59 | **69** | 8.51 | **99** | 12.16 |
| **33** | 0.69 | **70** | 6.00 | **100** | 27.24 |
| **34** | 1.37 | **71** | 3.09 | **101** | 8.73 |
| **35** | 1.49 | **72** | 9.40 | **102** | 24.32 |
| **36** | 1.44 | **73** | 6.70 | **103** | 9.87 |
| **37** | 1.59 | **74** | 2.20 | **104** | 10.48 |
| **38** | 1.51 | **75** | 3.09 | **105** | 25.22 |
| **39** | 1.53 | **76** | 4.96 | **106** | 10.48 |
| **40** | 0.49 | **77** | 9.00 | **108** | 3.64 |
| **43** | 1.71 | **77** | 9.00 | **109** | 11.54 |
| **44** | 0.78 | **78** | 23.33 | | |

According to Table 3, compared with the positive groups (TCP-25), the compounds of the present invention had obvious anti-inflammatory effects, the IC50 values of the compounds were significantly lower than those of the positive control groups, and activity multiples of the compounds were 1.04-27.24 times higher than those of the positive control groups.

Data processing: in order to more intuitively show the advantages of the compounds of the present invention relative to the positive control groups (TCP-25), data obtained from experimental groups comprising the negative control groups in which the compounds 1, 30, 78, 105 and 106 were only added with the 1 ×Loading buffer and the positive control groups (TCP-25) in which the compounds were only added with the LPS were calculated and analyzed according to the following formula, so as to obtain FIG. 11:

$$\text{Inhibition\%}=\left(1-\frac{\text{Experimental well signal value - Negative control mean value}}{\text{Positive control mean value - Negative control mean value}}\right)\times 100\%$$

According to FIG 3, compared with the positive groups, the compounds of the present invention had obvious anti-inflammatory effects, and the IC50 values of the compounds were significantly lower than those of the positive control groups.

The above have described the preferred embodiments of the present invention in detail, but the present invention

is not limited to the embodiments. Those skilled in the art may further make various equivalent modifications or substitutions without violating the spirit of the present invention, and these equivalent modifications or substitutions are all included in the scope defined by the claims of the present application.

## Claims

1. **1.** A polypeptide, comprising a sequence as shown in formula (III) below:

$$G\text{-}K\text{-}X^{10}\text{-}G\text{-}X^{11}\text{-}Y\text{-}X^{12}\text{-}X^{1}\text{-}X^{2}\text{-}X^{3}\text{-}X^{4}\text{-}L\text{-}X^{5}\text{-}K\text{-}W\text{-}I\text{-}X^{6}\text{-}K\text{-}X^{7}\text{-}X^{8}\text{-}X^{13}\text{-}X^{14}\text{-}X^{15}\text{-}G\text{-}X^{16} \quad \text{(III)}$$

or a pharmaceutically acceptable salt thereof, wherein:

$X^1$, $X^4$ and $X^5$ are respectively independently selected from K, dK, R, dR, and a derivative of K or R;
$X^2$, $X^3$, $X^6$, $X^{10}$, $X^{11}$, $X^{12}$, $X^{13}$, $X^{14}$, $X^{15}$ and $X^{16}$ are respectively independently selected from any amino acid;
$X^7$ is selected from V, L, or a d configuration of the amino acid and a derivative thereof; and
$X^8$ is selected from I, V, L, T, or the d configuration of the amino acid and the derivative thereof.

**2.** The polypeptide according to claim 1, wherein,

the $X^2$ is selected from V, L, T, K, W, E, or the d configuration of the amino acid and the derivative thereof;
the $X^3$ and the $X^{11}$ are respectively independently selected from F, Y, W, or the d configuration of the amino acid and the derivative thereof;
the $X^6$ is selected from Q, N, T, K, W, L, or the d configuration of the amino acid and the derivative thereof;
the $X^{10}$ is Y, F, W, or the d configuration of the amino acid and the derivative thereof; the $X^{12}$ is T, S, K, L, W, E, or the d configuration of the amino acid and the derivative thereof;
the $X^{13}$ and the $X^{16}$ are respectively independently selected from C, K, G, W, L, S, D, E, or the d configuration of the amino acid and the derivative thereof;
the $X^{14}$ is Q, N, T, K, W, L, or the d configuration of the amino acid and the derivative thereof; and
the $X^{15}$ is an optional amino acid, and is preferably Y, F, W, or the d configuration of the amino acid and the derivative thereof.

**3.** The polypeptide according to claim 1, wherein the $X^{13}$ and the $X^{16}$ are respectively independently selected from C or d-C.

**4.** The polypeptide according to claim 1, wherein the $X^{13}$ and the $X^{16}$ are linked into a ring through a bond; preferably, the bond is selected from a disulfide bond, an amide bond, a carbon-hydrogen bond, dithioamino $C_{1\text{-}6}$ alkyl, a thioether bond or an olefin thioether bond; and more preferably, the bond is one of the following structures:

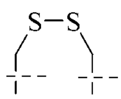, 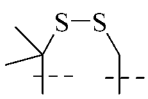, 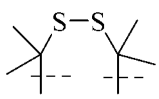, 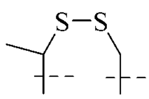, 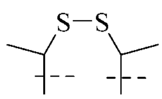, 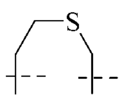, 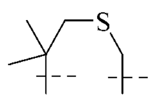,

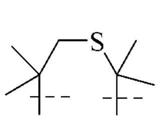, 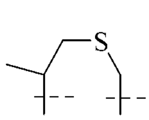, 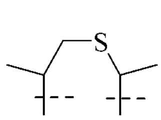, 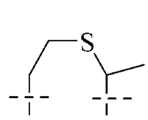, 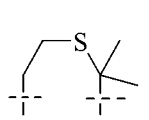, 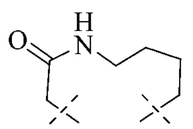,

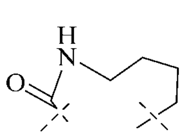, 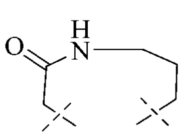, 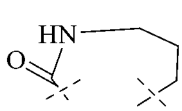, 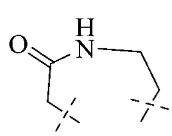, 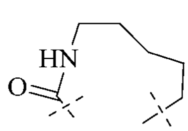, 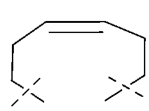,

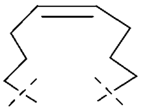, 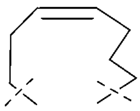, 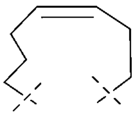, 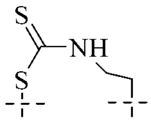 and 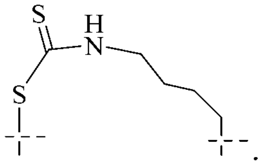.

5. The polypeptide according to claim 1, wherein the polypeptide is one of the following structures or a pharmaceutically acceptable salt thereof:

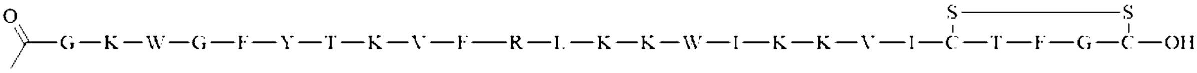

105

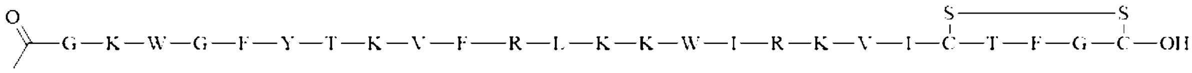

106

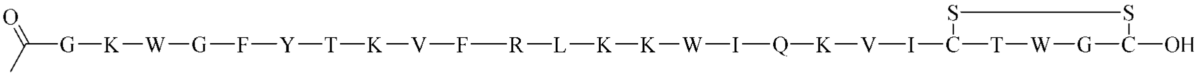

96

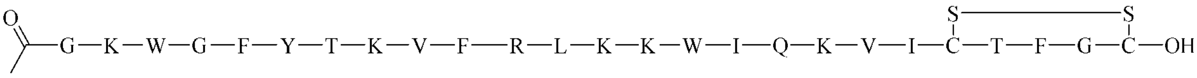

97

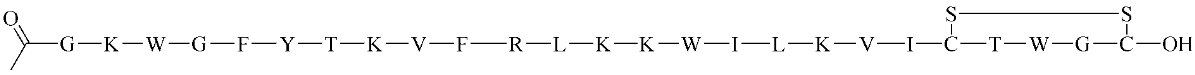

98

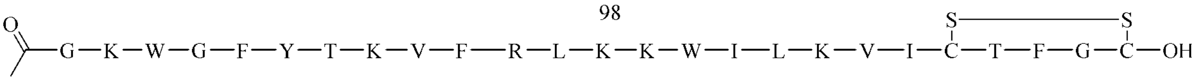

99

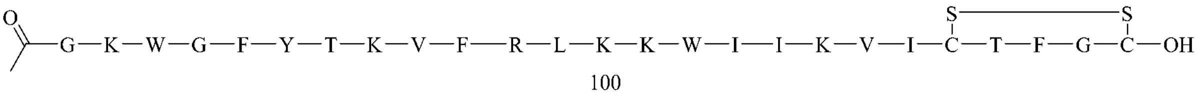

100

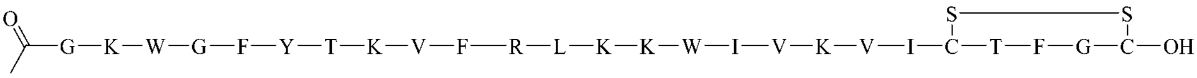

101

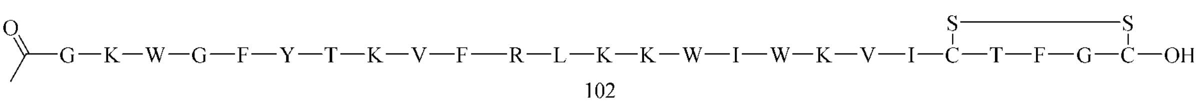

102

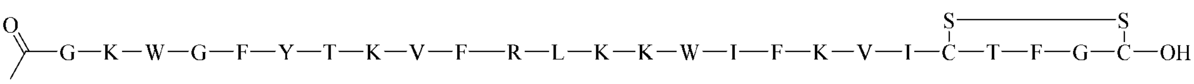

103

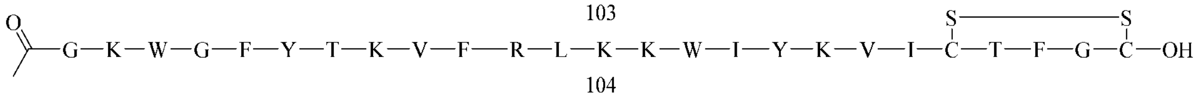

104

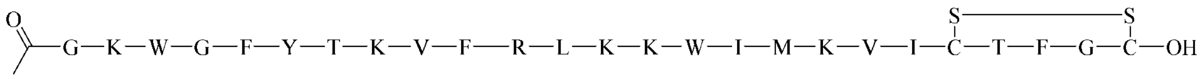

107

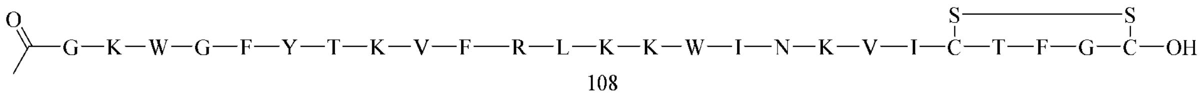

108

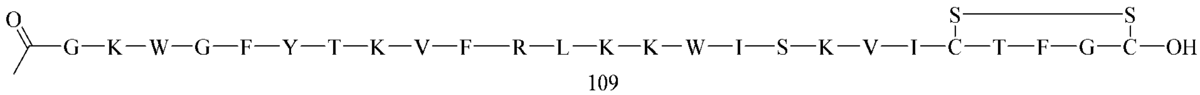

109

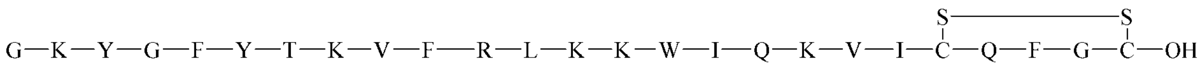

1

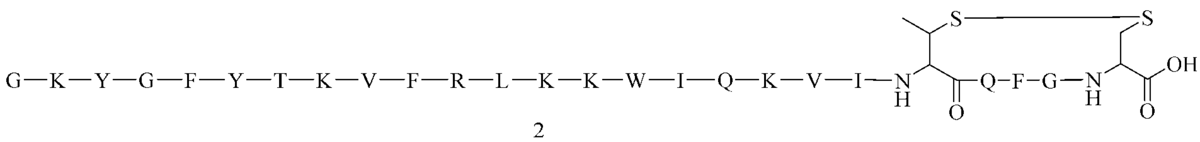

2

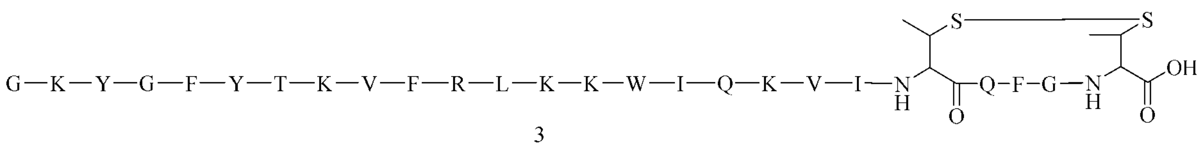

3

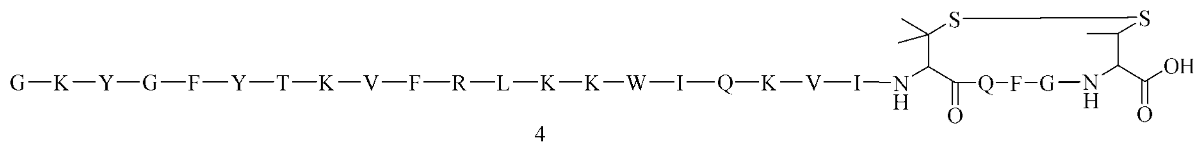

4

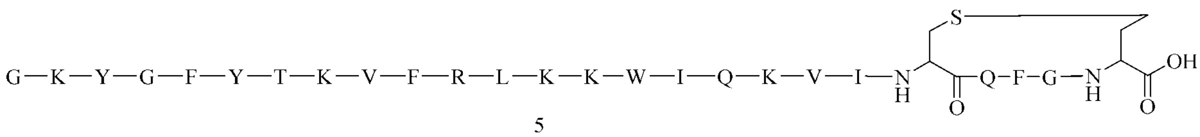

5

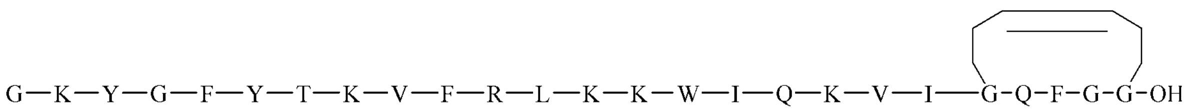

6

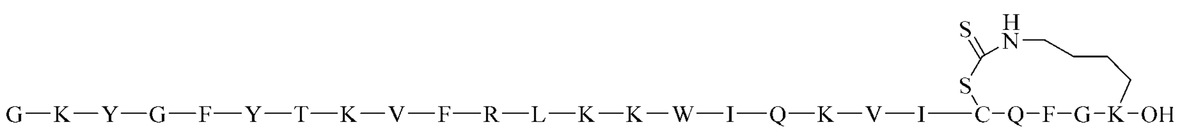

7

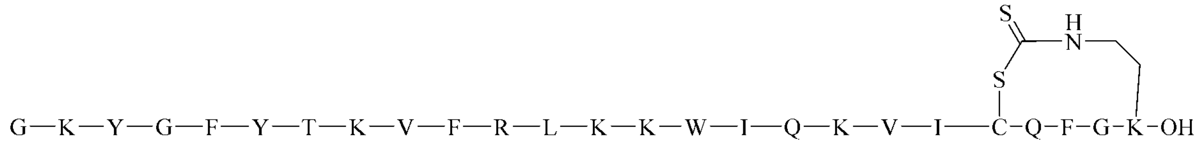
8

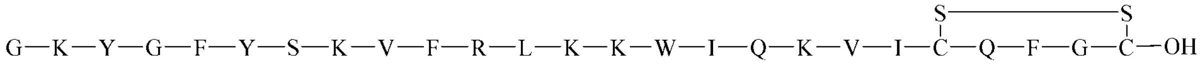
9

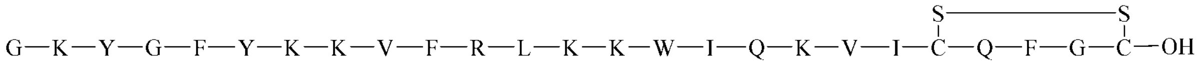
10

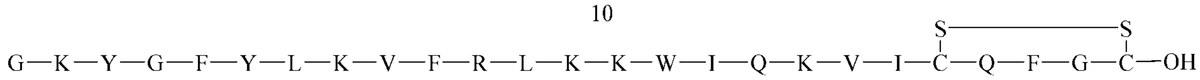
11

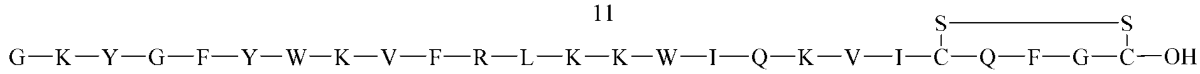
12

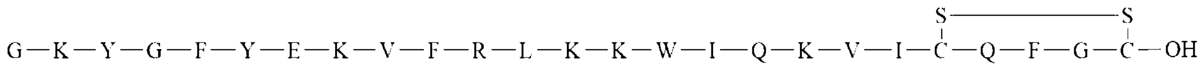
13

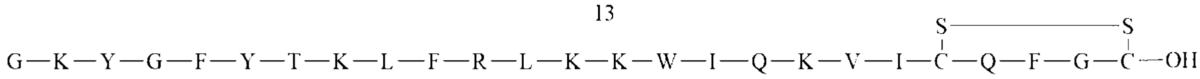
14

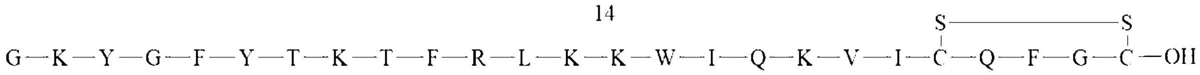
15

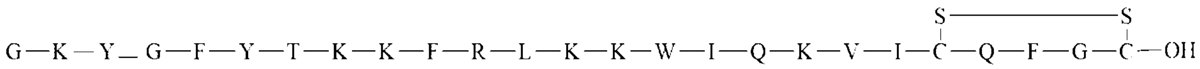
16

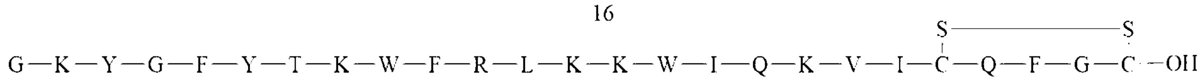
17

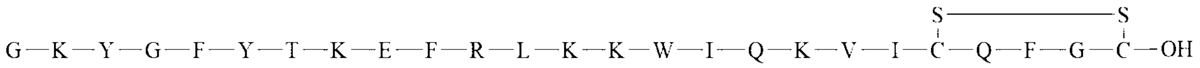
18

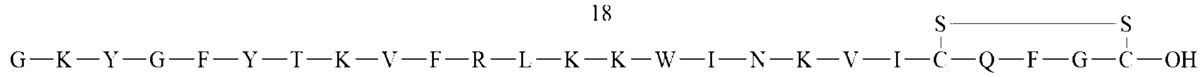
19

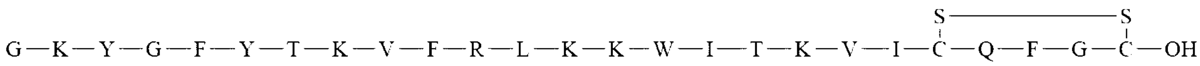
20

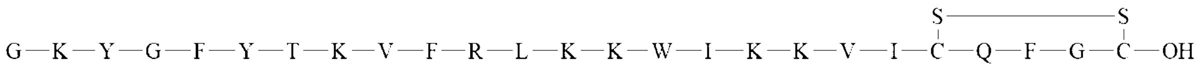
21

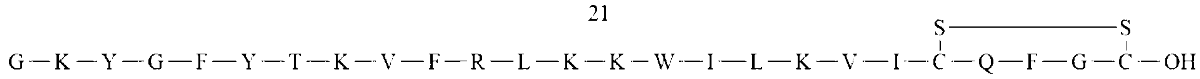
22

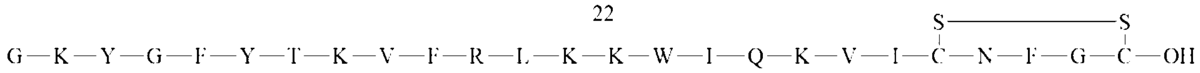
23

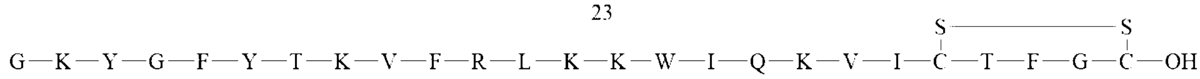
24

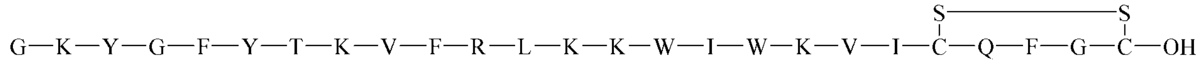

25

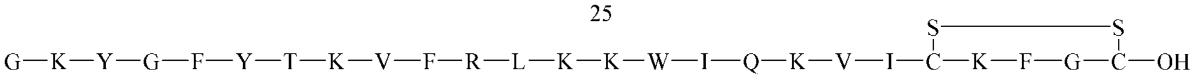

26

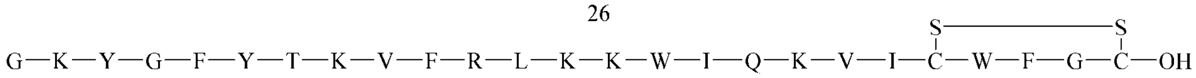

27

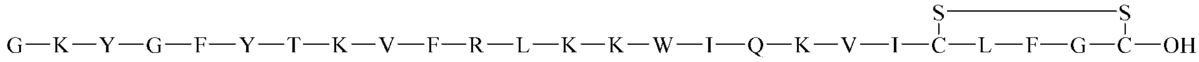

28

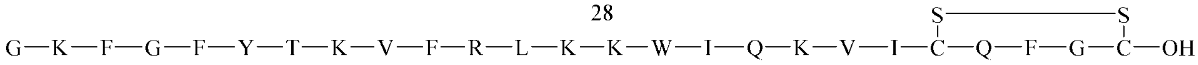

29

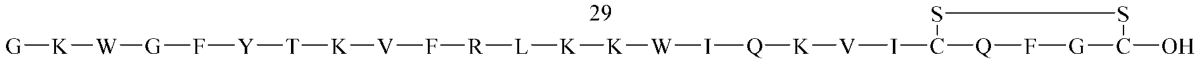

30

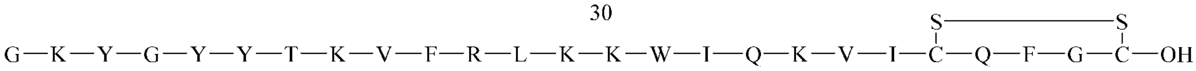

31

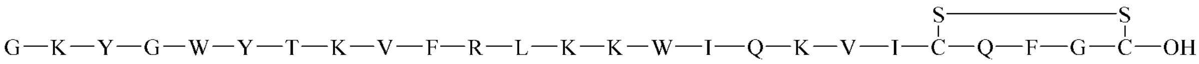

32

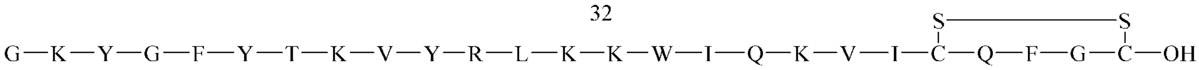

33

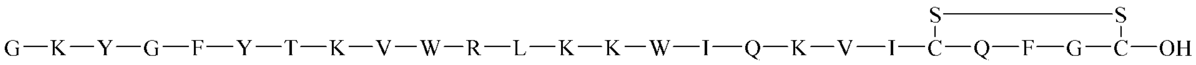

34

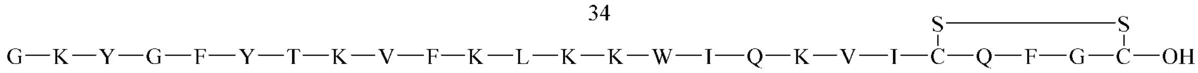

35

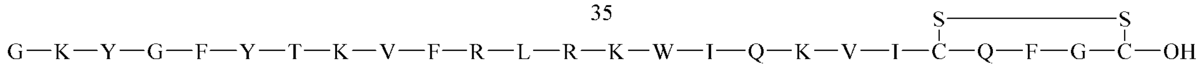

36

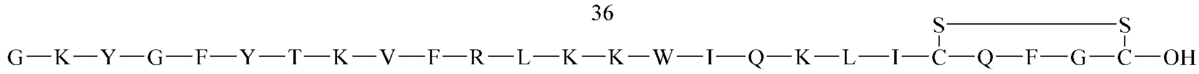

37

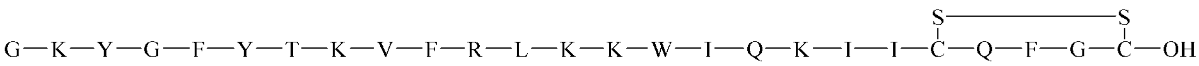

38

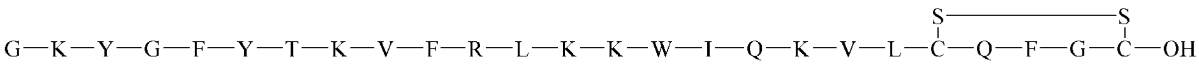

39

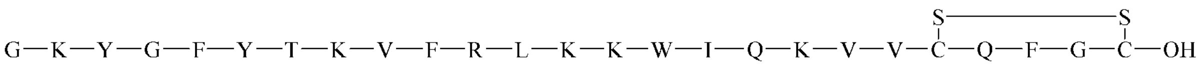

40

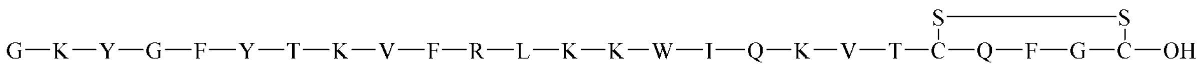

41

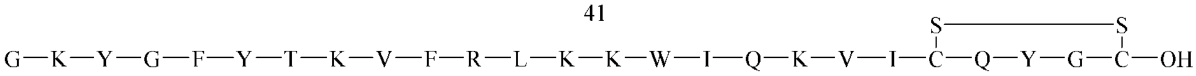

42

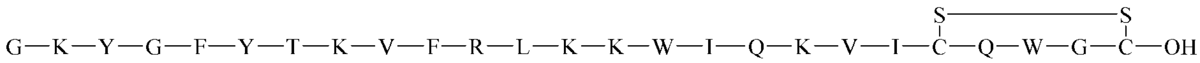

43

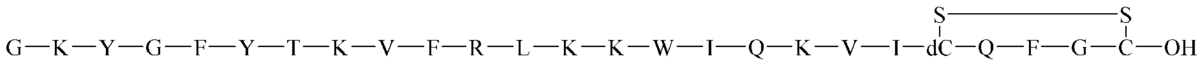

44

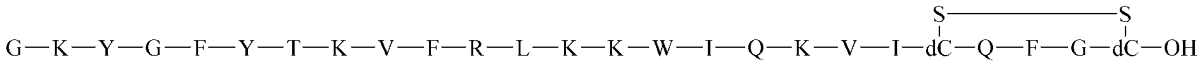

45

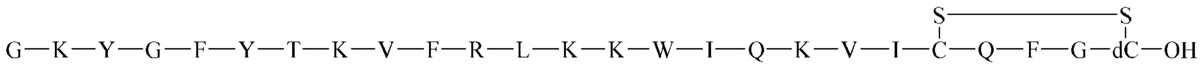

46

G—K—Y—G—F—Y—T—K—V—F—R—L—K—K—W—I—Q—K—V—I—K—Q—F—G—E—OH

47

G—K—Y—G—F—Y—T—K—V—F—R—L—K—K—W—I—Q—K—V—I—D—Q—F—G—K—OH

48

G—K—Y—G—F—Y—T—K—V—F—R—L—K—K—W—I—Q—K—V—I—K—Q—F—G—K—OH

49

G—K—Y—G—F—Y—T—K—V—F—R—L—K—K—W—I—Q—K—V—I—L—Q—F—G—E—OH

50

G—K—Y—G—F—Y—T—K—V—F—R—L—K—K—W—I—Q—K—V—I—W—Q—F—G—E—OH

51

G—K—Y—G—F—Y—T—K—V—F—R—L—K—K—W—I—Q—K—V—I—S—Q—F—G—E—OH

52

G—K—Y—G—F—Y—T—K—V—F—R—L—K—K—W—I—Q—K—V—I—D—Q—F—G—L—OH

53

G—K—Y—G—F—Y—T—K—V—F—R—L—K—K—W—I—Q—K—V—I—D—Q—F—G—W—OH

54

G—K—Y—G—F—Y—T—K—V—F—R—L—K—K—W—I—Q—K—V—I—D—Q—F—G—S—OH

55

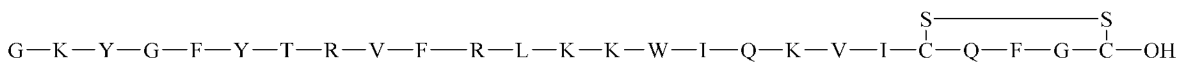

56

S———————S
G—K—W—G—W—Y—T—K—V—F—R—L—K—K—W—I—Q—K—V—I—C—Q—F—G—C—OH

57

S———————S
G—K—W—G—W—Y—W—K—V—F—R—L—K—K—W—I—Q—K—V—I—C—Q—F—G—C—OH

58

S———————S
G—K—W—G—F—Y—W—K—V—F—R—L—K—K—W—I—Q—K—V—I—C—Q—F—G—C—OH

59

S———————S
G—K—W—G—W—Y—W—R—V—F—R—L—K—K—W—I—Q—K—V—I—C—Q—F—G—C—OH

60

S———————S
G—K—W—G—W—Y—W—R—L—F—R—L—K—K—W—I—Q—K—V—I—C—Q—F—G—C—OH

61

S———————S
G—K—W—G—W—Y—W—R—L—W—R—L—K—K—W—I—Q—K—V—I—C—Q—F—G—C—OH

62

S———————S
G—K—W—G—W—Y—W—R—L—W—K—L—K—K—W—I—Q—K—V—I—C—Q—F—G—C—OH

63

S———————S
G—K—W—G—W—Y—W—R—L—W—K—L—R—K—W—I—Q—K—V—I—C—Q—F—G—C—OH

64

S———————S
G—K—W—G—W—Y—W—R—L—W—K—L—R—K—W—I—W—K—V—I—C—Q—F—G—C—OH

65

S———————S
G—K—W—G—W—Y—W—R—L—W—K—L—R—K—W—I—L—K—V—I—C—Q—F—G—C—OH

66

S———————S
G—K—W—G—W—Y—W—R—L—W—K—L—R—K—W—I—W—K—L—I—C—Q—F—G—C—OH

67

S———————S
G—K—W—G—W—Y—W—R—L—W—K—L—R—K—W—I—W—K—L—L—C—Q—F—G—C—OH

68

S———————S
G—K—W—G—W—Y—W—R—L—W—K—L—R—K—W—I—W—K—L—L—C—T—F—G—C—OH

69

S———————S
G—K—W—G—W—Y—W—R—L—W—K—L—R—K—W—I—W—K—L—L—C—T—W—G—C—OH

70

S———————S
G—K—W—G—W—Y—W—R—L—W—K—L—R—K—W—I—L—K—L—L—C—T—W—G—C—OH

71

S———————S
G—K—W—G—W—Y—W—R—L—W—K—L—R—K—W—I—L—K—L—L—C—W—W—G—C—OH

72

S———————S
G—K—W—G—W—Y—W—R—K—W—K—L—R—K—W—I—L—K—L—L—C—T—W—G—C—OH

73

S———————S
G—K—W—G—F—Y—T—R—V—F—R—L—K—K—W—I—Q—K—V—I—C—Q—W—G—C—OH

74

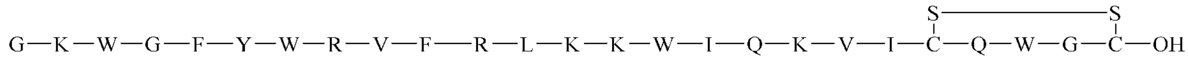

75

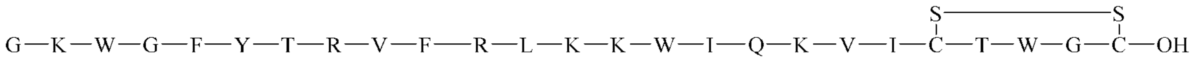

76

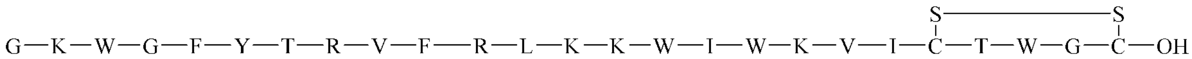

77

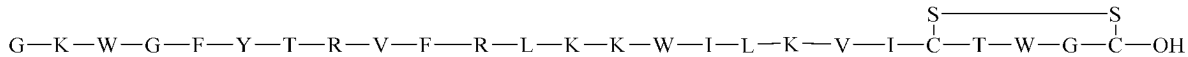

78

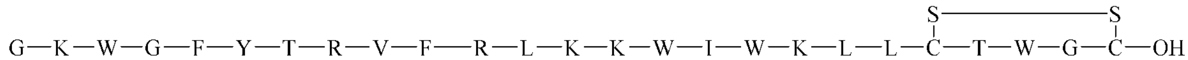

79

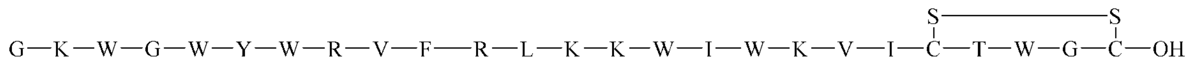

80

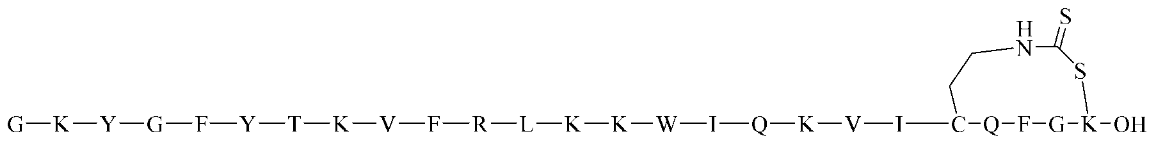

81

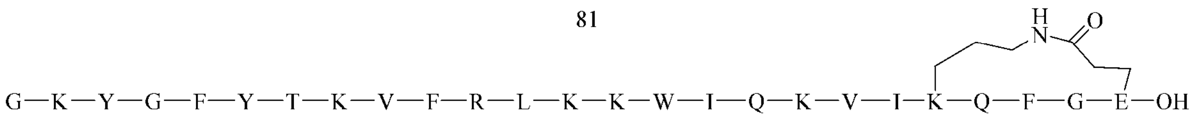

82

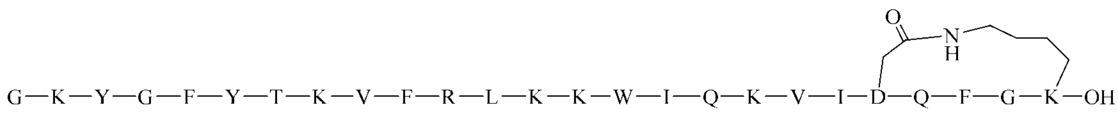

83

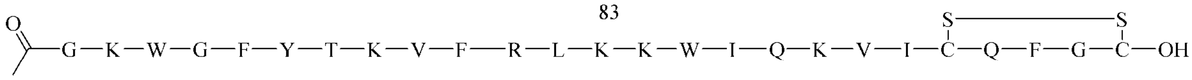

84

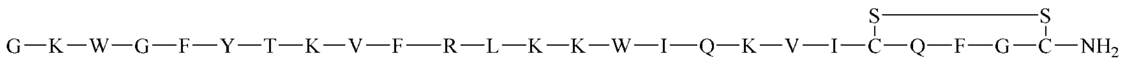

85

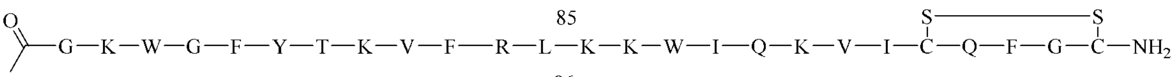

86

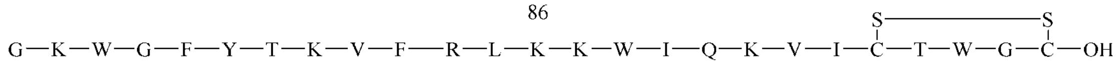

87

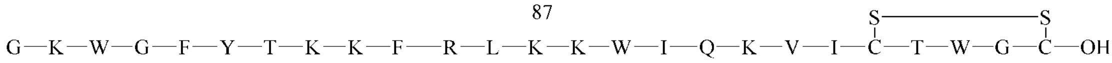

88

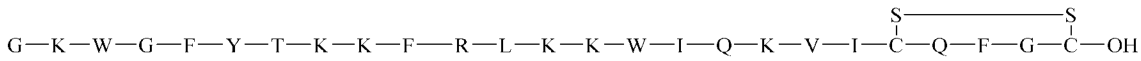

89

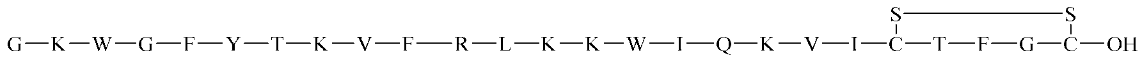

90

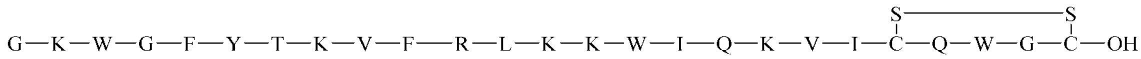

91

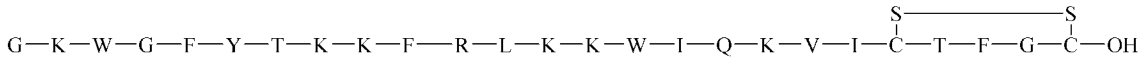

92

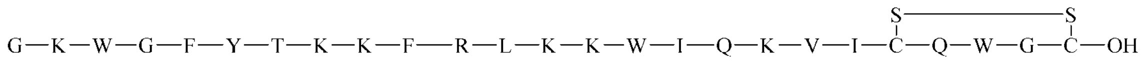

93

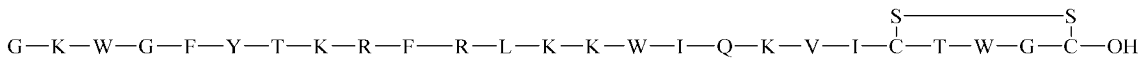

94

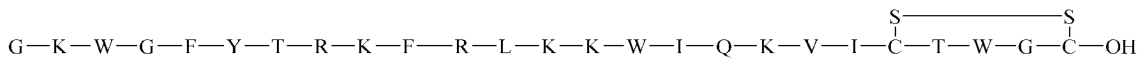

95

.

6. A polypeptide, selected from one of the following amino acid sequences: SEQ ID No. 1-SEQ ID No. 99.

7. A pharmaceutical composition, comprising the polypeptide according to any one of claims 1 to 6.

8. The pharmaceutical composition according to claim 7, further comprising at least one of a pharmaceutically acceptable carrier, an excipient, a diluent, an adjuvant or an intermedium.

9. A use of the polypeptide according to any one of claims 1 to 6 or the pharmaceutical composition according to any one of claims 7 to 8 in preparation of a drug for preventing, managing, treating or alleviating a bacterial infectious disease and/or an inflammatory disease.

10. The use according to claim 9, wherein the bacterial infectious disease and/or the inflammatory disease is any one of skin infection, skin wound infection, burn infection, scald infection, pneumonia, tuberculosis, diabetic foot, tonsillitis, bacillary dysentery, meningitis, scarlet fever, pharyngolaryngitis, bronchitis, gastritis, appendicitis, glomerulonephritis, endocarditis, pericarditis, cystitis, pelvic inflammation and cervicitis.

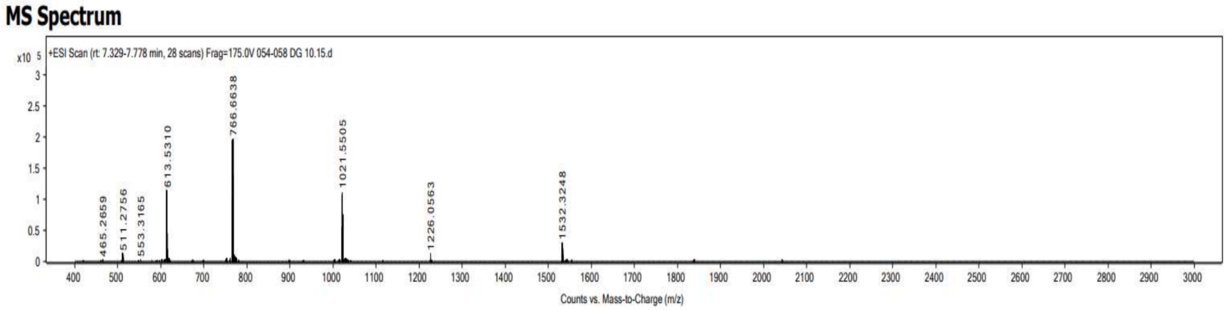


FIG. 1

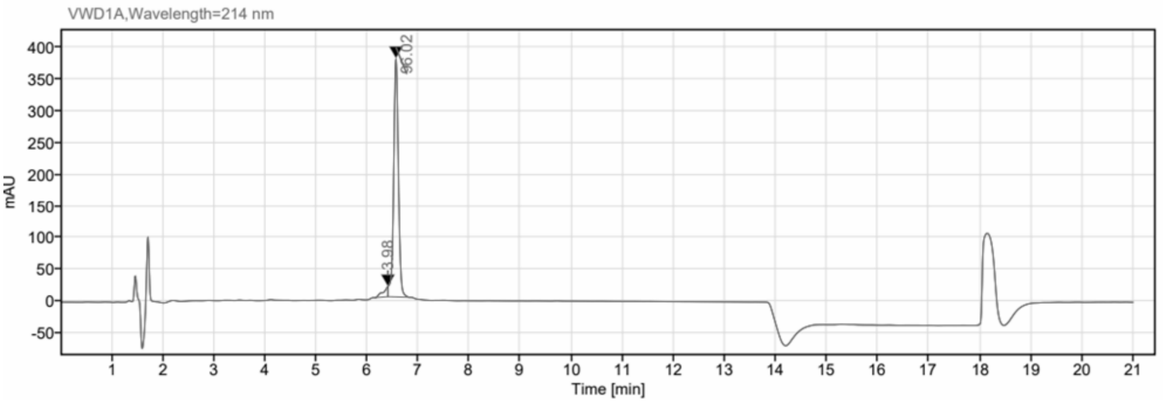


FIG. 2

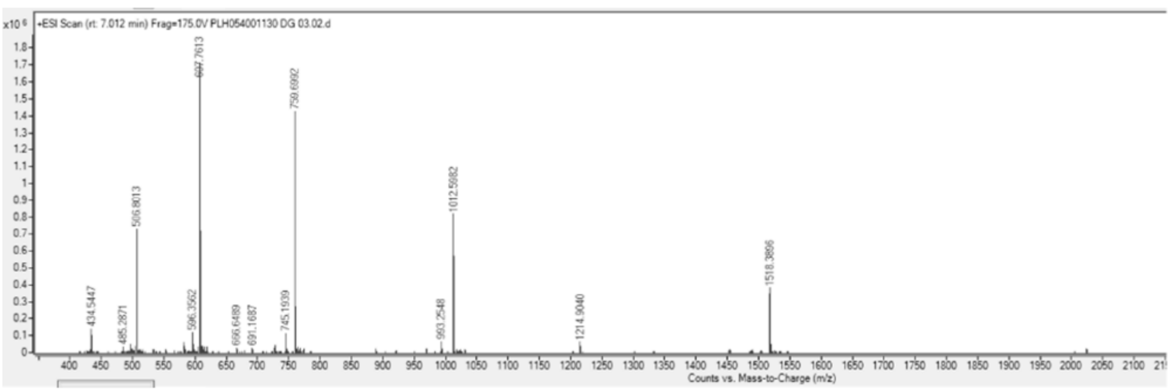


FIG. 3

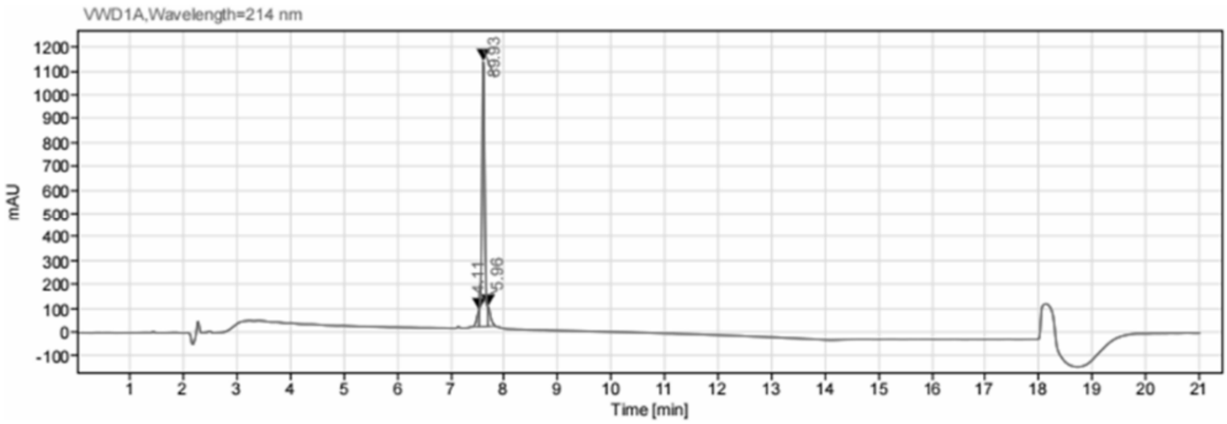


FIG. 4

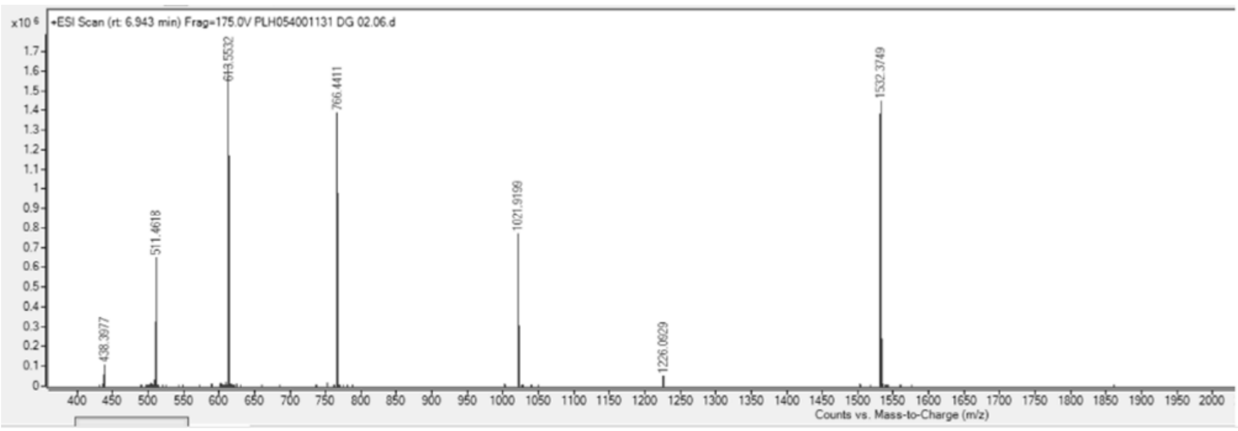


FIG. 5

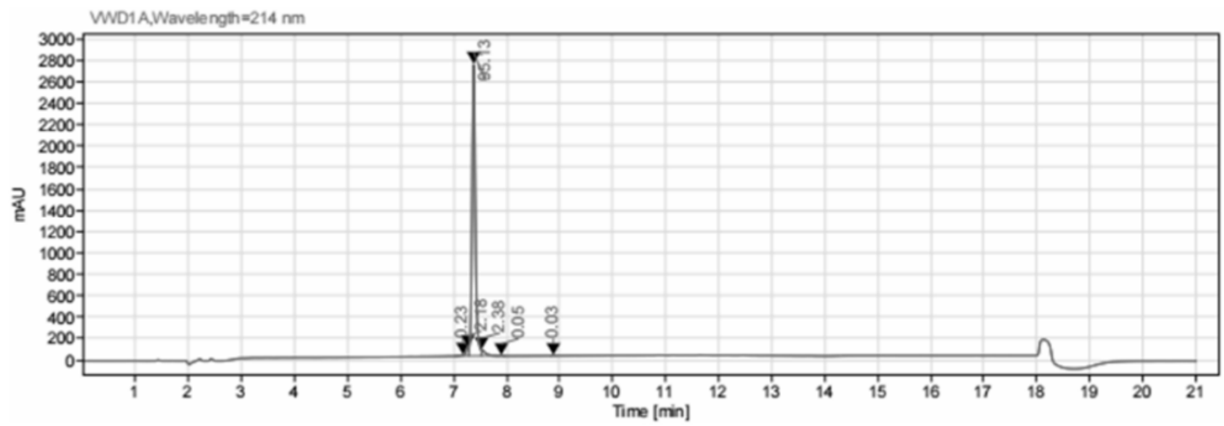


FIG. 6

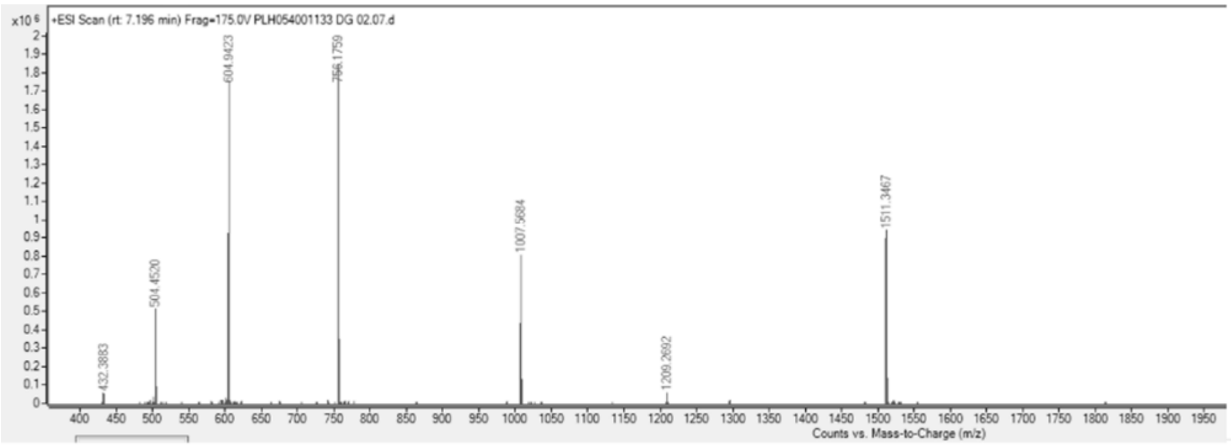


FIG. 7

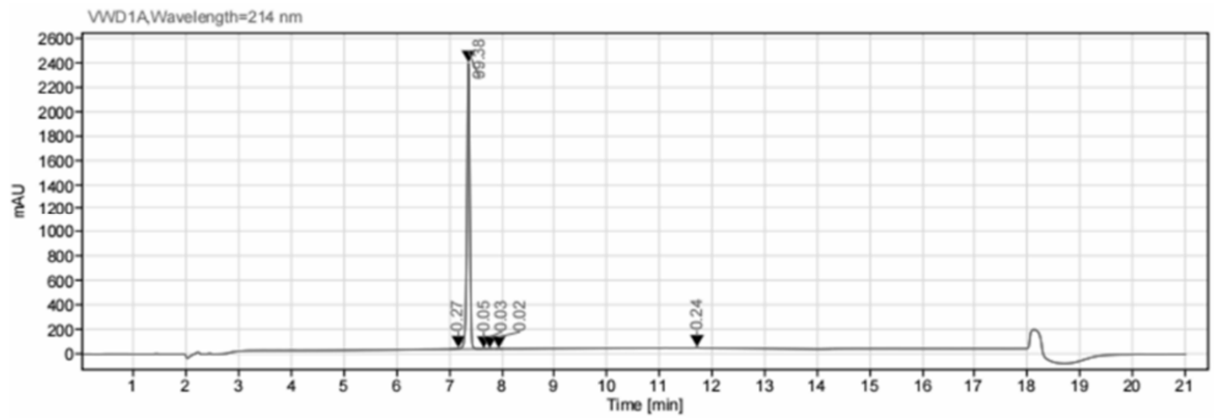


FIG. 8

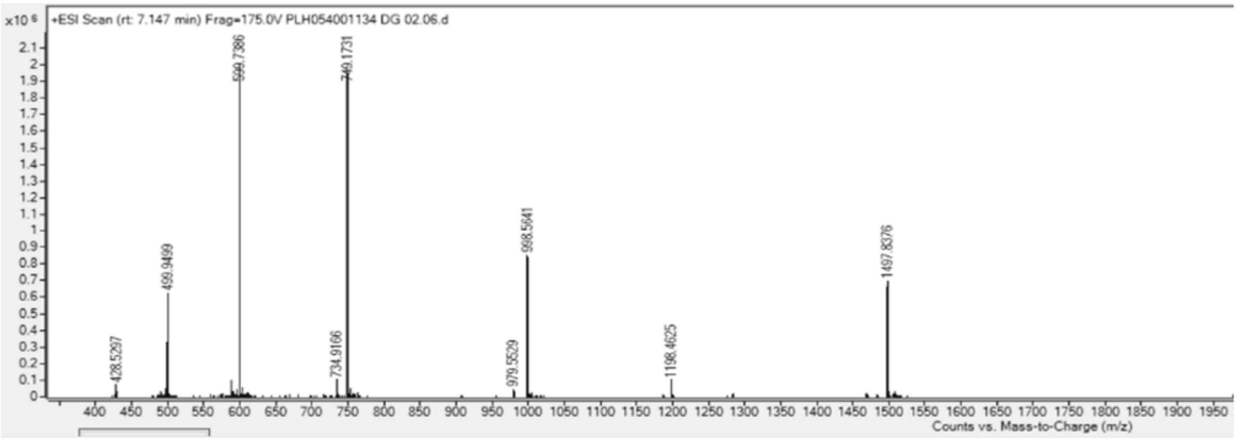


FIG. 9

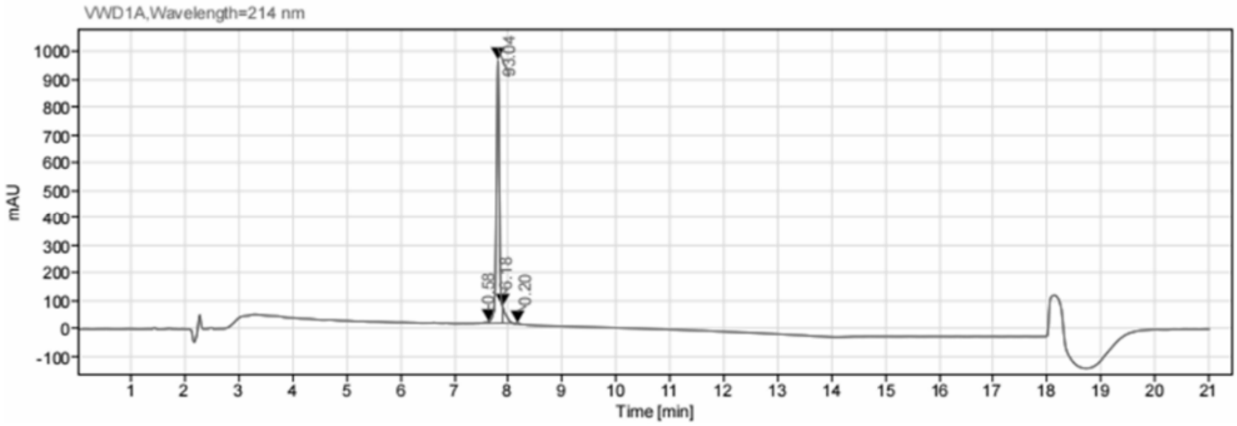


FIG. 10

**Inhibitory effect of polypeptide on TLR4 cells activated by LPS**

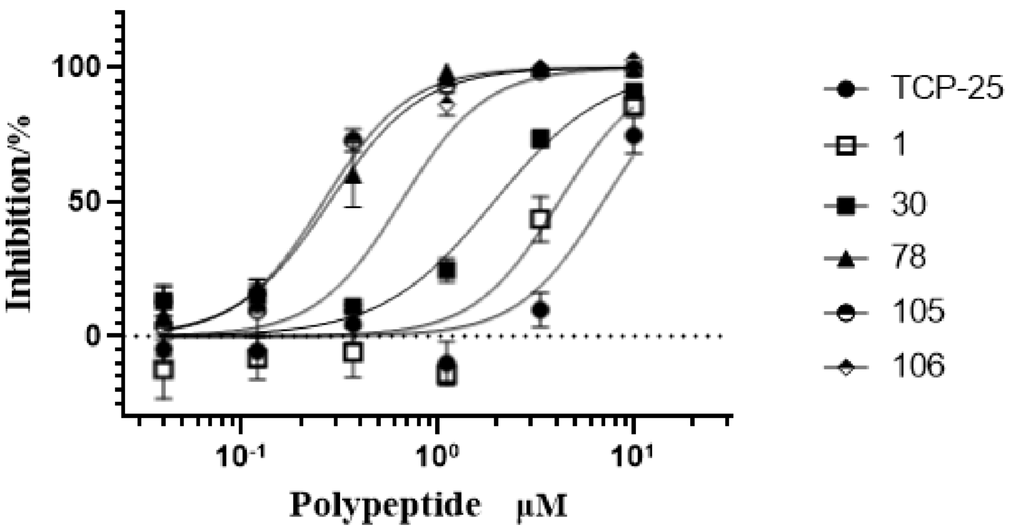


FIG. 11

# INTERNATIONAL SEARCH REPORT

International application No.
**PCT/CN2024/074412**

## A. CLASSIFICATION OF SUBJECT MATTER

C12N 9/74(2006.01)i; C07K 7/04(2006.01)i; C07K 14/00(2006.01)i; A61K 38/16(2006.01)i; A61P 31/04(2006.01)i

According to International Patent Classification (IPC) or to both national classification and IPC

## B. FIELDS SEARCHED

Minimum documentation searched (classification system followed by classification symbols)

IPC: C12N C07K A61K A61P

Documentation searched other than minimum documentation to the extent that such documents are included in the fields searched

Electronic data base consulted during the international search (name of data base and, where practicable, search terms used)

CNABS; CNTXT; CJFD; CNKI; WPABS; WPABSC; ENTXTC; ENTXT; DWPI; ISI Web of Science; GenBank; STNext; 中国专利生物序列检索系统, Chinese Patent Biological Sequence Retrieval System: 凝血酶, TCP, TCP-25, 衍生, 突变, 抗菌, 抗炎, 序列1-99, 化合物1-109, thrombin, thrombin-derived C-terminal peptides, mutation, antibacterial, antiinflammatory

## C. DOCUMENTS CONSIDERED TO BE RELEVANT

| Category* | Citation of document, with indication, where appropriate, of the relevant passages | Relevant to claim No. |
|---|---|---|
| A | WO 2021260164 A1 (IN2CURE AB) 30 December 2021 (2021-12-30)<br>description, pages 9-13 | 1-10 |
| A | CN 101535479 A (CHISSO CORP. et al.) 16 September 2009 (2009-09-16)<br>claims 1-10 | 1-10 |
| A | US 2009143299 A1 (SCHMIDTCHEN ARTUR et al.) 04 June 2009 (2009-06-04)<br>claims 1-16 | 1-10 |
| A | US 2013052258 A1 (KALLE MARTINA et al.) 28 February 2013 (2013-02-28)<br>claims 1-79 | 1-10 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 April 2024** | **24 April 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/074412**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/074412**

| Patent document cited in search report | Publication date (day/month/year) | Patent family member(s) | Publication date (day/month/year) |
|---|---|---|---|
| WO 2021260164 A1 | 30 December 2021 | EP 4171615 A1 | 03 May 2023 |
| | | US 2023218727 A1 | 13 July 2023 |
| CN 101535479 A | 16 September 2009 | CA 2669641 A1 | 22 May 2008 |
| | | JPWO 2008059917 A1 | 04 March 2010 |
| | | EP 2399994 A1 | 28 December 2011 |
| | | US 2010040597 A1 | 18 February 2010 |
| | | US 8088372 B2 | 03 January 2012 |
| | | RU 2009122486 A | 20 December 2010 |
| | | EP 2093290 A1 | 26 August 2009 |
| | | EP 2093290 A4 | 24 March 2010 |
| | | AU 2007320353 A1 | 22 May 2008 |
| | | KR 20090081388 A | 28 July 2009 |
| | | WO 2008059917 A1 | 22 May 2008 |
| US 2009143299 A1 | 04 June 2009 | CA 2637221 A1 | 16 August 2007 |
| | | JP 2009526046 A | 16 July 2009 |
| | | US 8076286 B2 | 13 December 2011 |
| | | WO 2007091959 A1 | 16 August 2007 |
| | | EP 1987056 A1 | 05 November 2008 |
| | | EP 1987056 B1 | 25 July 2012 |
| US 2013052258 A1 | 28 February 2013 | US 8735353 B2 | 27 May 2014 |
| | | EP 2480567 A2 | 01 August 2012 |
| | | EP 2480567 B1 | 10 August 2016 |
| | | GB 0916576 D0 | 28 October 2009 |
| | | WO 2011036442 A2 | 31 March 2011 |
| | | WO 2011036442 A3 | 30 June 2011 |
| | | WO 2011036443 A2 | 31 March 2011 |
| | | WO 2011036443 A3 | 06 October 2011 |
| | | WO 2011036443 A9 | 02 February 2012 |
| | | DK 2480567 T3 | 28 November 2016 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning: A Laboratory Manual. Cold Spring Harbor Laboratory, 2001
- **AUSUBEL et al.** Current Protocols in Molecular Biology. John Wiley and Sons, 1999
- **SMITH** ; **WATERMAN**. *J. Theor. Biol.*, 1981
- **NEEDLEMAN** ; **WUNSCH**. *J. Mol. Biol*, 1972
- **PEARSON** ; **LIPMAN**. *Proc. Natl. Acad. Sci. U.S.A.*, 1988
- Remington's Pharmaceutical Sciences. Mack Publishing Company, 1985
- **STAHL** ; **WERMUTH**. Handbook of Pharmaceutical Salts: Properties, Selection, and Use. Wiley-VCH, 2002